# EUROPEAN PATENT SPECIFICATION

(11) **EP 3 447 139 B1**
(45) Date of publication and mention of the grant of the patent: **08.06.2022**
(21) Application number: 17786061.6
(22) Date of filing: 21.04.2017
(51) Int. Cl.: C12N 15/10, C12N 9/22, C12N 9/78

(54) **METHOD FOR INCREASING MUTATION INTRODUCTION EFFICIENCY IN GENOME SEQUENCE MODIFICATION TECHNIQUE, AND MOLECULAR COMPLEX TO BE USED THEREFOR**
VERFAHREN ZUR ERHÖHUNG DER MUTATIONSEINFÜHRUNGSEFFIZIENZ IN EINEM GENOMSEQUENZMODIFIZIERUNGSVERFAHREN UND MOLEKULARER KOMPLEX ZUR VERWENDUNG DAFÜR
PROCÉDÉ D'AUGMENTATION DE L'EFFICACITÉ D'INTRODUCTION D'UNE MUTATION DANS UNE TECHNIQUE DE MODIFICATION D'UNE SÉQUENCE GÉNOMIQUE, ET COMPLEXE MOLÉCULAIRE À UTILISER À CET EFFET

(30) Priority: 21.04.2016 JP 2016085631
(43) Date of publication of application: 27.02.2019
(73) Proprietor: National University Corporation Kobe University, Hyogo 657-8501 (JP)
(72) Inventor: NISHIDA, Keiji, Kobe-shi Hyogo 657-8501 (JP); KONDO, Akihiko, Kobe-shi Hyogo 657-8501 (JP); ARAZOE, Takayuki, Kobe-shi Hyogo 657-8501 (JP); SHIMATANI, Zenpei, Kobe-shi Hyogo 657-8501 (JP)
(74) Representative: J A Kemp LLP
(86) International application number: PCT/JP2017/016105
(87) International publication number: WO 2017/183724

(56) References cited:
- EP-A1- 3 216 867
- EP-A1- 3 382 019
- WO-A1-2015/133554
- WO-A1-2016/072399
- WO-A1-2017/090761
- WO-A2-2017/070632
- K. NISHIDA ET AL: "SUPPLEMENTARY MATERIAL for : Targeted nucleotide editing using hybrid prokaryotic and vertebrate adaptive immune systems", SCIENCE HTTPS://SCIENCE.SCIENCEMAG.ORG/CONTENT/SCI /SUPPL/2016/08/03/SCIENCE.AAF8729.DC1/NISH IDA-SM.PDF, vol. 353, no. 6305, 4 August 2016 (2016-08-04), pages 1-35, XP55482712, US ISSN: 0036-8075, DOI: 10.1126/science.aaf8729 Retrieved from the Internet: URL:https://science.sciencemag.org/content /sci/suppl/2016/08/03/science.aaf8729.DC1/ Nishida-SM.pdf> [retrieved on 2019-08-21]
- KUSCU CEM ET AL: "CRISPR-Cas9-AID base editor is a powerful gain-of-function screening tool.", NATURE METHODS 29 11 2016, vol. 13, no. 12, 29 November 2016 (2016-11-29), pages 983-984, XP002793705, ISSN: 1548-7105
- YUNQING MA ET AL: "Targeted AID-mediated mutagenesis (TAM) enables efficient genomic diversification in mammalian cells", NATURE METHODS, vol. 13, no. 12, 1 December 2016 (2016-12-01), pages 1029-1035, XP55573969, New York ISSN: 1548-7091, DOI: 10.1038/nmeth.4027
- NISHIDA K ET AL.: 'Targeted nucleotide editing using hybrid prokaryotic and vertebrate adaptive immune systems' SCIENCE, [Online] vol. 353, 04 August 2016, page AAF8729, XP055367833 Retrieved from the Internet: <URL:http:// science .sciencemag. org/content/353/6305/aaf8729.1ong> [retrieved on 2017-07-06]
- ONTICELLO SG ET AL.: 'Evolution of the AID/ APOBEC family of polynucleotide (deoxy)cytidine deaminases' MOL. BIOL. EVOL. vol. 22, no. 2, 2005, pages 367 - 377, XP002667007
- KOMOR AC ET AL.: 'Programmable editing of a target base in genomic DNA without double- stranded DNA cleavage' NATURE, [Online] vol. 533, no. 7603, 20 April 2016, pages 420 - 424, XP055343871 Retrieved from the Internet: <URL:http://www. nature .com/ nature /journal/v533/n7603/full/ nature 17946. html> [retrieved on 2017-07-06]
- ROGOZIN IB ET AL.: 'Evolution and diversification of lamprey antigen receptors: evidence for involvement of an AID-APOBEC family cytosine deaminase' NAT. IMMUNOL. vol. 8, no. 6, 2007, pages 647 - 656, XP002503283
- KITAMURA K ET AL.: 'Uracil DNA glycosylase counteracts APOBEC3G-induced hypermutation of hepatitis B viral genomes: excision repair of covalently closed circular DNA' PLOS PATHOG., [Online] vol. 9, no. 5, 16 May 2013, page EL003361, XP055432854 Retrieved from the Internet: <URL:https://www. ncbi.nlm.nih.gov/pubmed/23696735> [retrieved on 2016-07-10]

## Description

### [Technical Field]

The present invention relates to a method for improving mutation introduction efficiency of a genome sequence modification technique that enables modification of a nucleic acid base in a particular region of a genome without cleaving double-stranded DNA, i.e., with no cleavage or single strand cleavage, or inserting a foreign DNA fragment, and a complex of a nucleic acid sequence-recognizing module, a nucleic acid base converting enzyme and a base excision repair inhibitor to be used therefor.

### [Background Art]

In recent years, genome editing is attracting attention as a technique for modifying the object gene and genome region in various species. Conventionally, as a method of genome editing, a method utilizing an artificial nuclease comprising a molecule having a sequence-independent DNA cleavage ability and a molecule having a sequence recognition ability in combination has been proposed (non-patent document 1).

For example, a method of performing recombination at a target gene locus in DNA in a plant cell or insect cell as a host, by using a zinc finger nuclease (ZFN) wherein a zinc finger DNA binding domain and a non-specific DNA cleavage domain are linked (patent document 1), a method of cleaving or modifying a target gene in a particular nucleotide sequence or a site adjacent thereto by using TALEN wherein a transcription activator-like (TAL) effector which is a DNA binding module that the plant pathogenic bacteria Xanthomonas has, and a DNA endonuclease are linked (patent document 2), a method utilizing CRISPR-Cas9 system wherein DNA sequence CRISPR (Clustered Regularly interspaced short palindromic repeats) that functions in an acquired immune system possessed by eubacterium and archaebacterium, and nuclease Cas (CRISPR-associated) protein family having an important function along with CRISPR are combined (patent document 3) and the like have been reported. Furthermore, a method of cleaving a target gene in the vicinity of a particular sequence, by using artificial nuclease wherein a PPR protein constituted to recognize a particular nucleotide sequence by a continuation of PPR motifs each consisting of 35 amino acids and recognizing one nucleic acid base, and nuclease are linked (patent document 4) has also been reported.

These genome editing techniques basically presuppose double-stranded DNA breaks (DSB). However, since they include unexpected genome modifications, side effects such as strong cytotoxicity, chromosomal rearrangement and the like occur, and they have common problems of impaired reliability in gene therapy, extremely small number of surviving cells by nucleotide modification, and difficulty in genetic modification itself in primate ovum and unicellular microorganisms.

On the other hand, as a method for performing nucleotide modification without accompanying DSB, the present inventors reported that, in the CRISPR-Cas system wherein at least one DNA cleavage ability of Cas is inactivated, a genome sequence was successfully modified without accompanying DSB and by nucleobase conversion in a region containing a specific DNA sequence. In the system, they used deaminase that catalyzes a deamination reaction, which was linked to a molecule having a DNA sequence recognition ability (patent document 5). According to this genome editing technique, since the technique does not involve insertion of foreign DNA or cleavage of DNA double strand, it is superior in safety, and the range of mutation introduction can theoretically be set widely from a single base pinpoint to several hundred bases. However, there was a problem that efficiency of mutation introduction is low as compared to genome editing technique using Cas9 having normal DNA cleaving ability.

In genome editing technique, moreover, a method for enhancing the efficiency of mutation introduction by shifting the culture temperature of the cell to a low temperature has not been reported. In addition, there is no report teaching that the activity of Petromyzon marinus-derived PmCDA1 (Petromyzon marinus cytosine deaminase 1), which is one kind of deaminase, is enhanced when the temperature is lower than about 37°C which is the optimal temperature of general enzymes.

Nishida K et al. (SCIENCE, vol. 353, 4 August 2016, page AAF8729 and Supplementary Material) reports targeted nucleotide editing using hybrid prokaryotic and vertebrate adaptive immune systems.

WO 2017/070632 A1 discloses nucleobase editors using fusion proteins of cas9 and uses thereof.

WO 2017/090761 A1 discloses a method for converting monocot plant genome sequence in which nucleic acid base in targeted DNA sequence is specifically converted, and molecular complex used therein.

Onticello SG et al, (Mol. Biol. Evol., vol. 22, no. 2, 2005, pages 367-377) reports evolution of the AID/APOBEC family of polynucleotide (deoxy) cytidine deaminases.

Komor AC et al. (Nature, vol. 533, no. 7603, 20 April 2016, pages 420-424) reports programmable editing of a target base in genomic DNA without double-stranded DNA cleavage.

### [Document List]

### [Patent documents]

patent document 1: JP-B-4968498
patent document 2: National Publication of International Patent Application No. 2013-513389
patent document 3: National Publication of International Patent Application No. 2010-519929
patent document 4: JP-A-2013-128413
patent document 5: WO 2015/133554

### [non-patent document]

non-patent document 1: Kelvin M Esvelt, Harris H Wang (2013) Genome-scale engineering for systems and synthetic biology, Molecular Systems Biology 9: 641

### [SUMMARY OF THE INVENTION]

### [Problems to be Solved by the Invention]

An object of the present invention is to provide a genome editing method for improving mutation introduction efficiency by modifying nucleic acid bases of a particular sequence of a gene by not cleaving double-stranded DNA or cleaving single strand, and a complex therefor of a nucleic acid sequence-recognizing module, a nucleic acid base converting enzyme, and a base excision repair inhibitor.

### [Means of Solving the Problems]

The present inventors searched for the development of a method for improving the mutation introduction efficiency in the genome editing technique using a nucleic acid base converting enzyme. In the development of a method for improving mutation introduction efficiency, in general, the focus is placed on a method for increasing the nucleic acid base converting ability by artificially mutating a nucleic acid base converting enzyme or replacing same with other enzyme, or a method for increasing the nucleic acid recognizing ability of a nucleic acid sequence-recognizing module and the like. The present inventors changed these general ideas and assumed that, in the genome editing technique, one of the causes of the low mutation introduction efficiency might be that the mechanism of base excision repair by DNA glycosylase or the like works at the site where the base was converted by the nucleic acid base converting enzyme and the introduced mismatch is repaired. They then had an idea that the mutation introduction efficiency might be increased by inhibiting proteins acting on the base excision repair mechanisms. Thus, the present inventors coexpressed a uracil DNA glycosylase inhibitor (Ugi) that inhibits repair of deaminated bases, and found that the mutation introduction efficiency was strikingly improved.

PmCDA1, which is one kind of nucleic acid base converting enzyme, is derived from Petromyzon marinus, a poikilothermic animal. Thus, they assumed that the optimal temperature for the enzyme activity of PmCDA1 might be lower than about 37°C, which is the optimal temperature for general enzymes, and had an idea that the enzyme activity might be enhanced by adjusting the culture temperature. In an attempt to enhance the enzyme activity of PmCDA1, therefore, they cultured the cells transfected with PmCDA1 temporarily at a low temperature and found that the mutation introduction efficiency was improved.

The present inventor have conducted further studies based on these findings and completed the present invention.

Therefore, the present invention is as described below.
[1] A method of modifying a targeted site of a double-stranded DNA, comprising a step of introducing a complex wherein a nucleic acid sequence-recognizing module that specifically binds to a target nucleotide sequence in a given double-stranded DNA and PmCDA1 are bonded, into a mammalian cell containing the double-stranded DNA, and culturing the cell at a temperature of 20°C to 35°C at least temporarily to convert one or more nucleotides in the targeted site to other one or more nucleotides or delete one or more nucleotides, or insert one or more nucleotides into said targeted site, without cleaving at least one strand of said double-stranded DNA in the targeted site,
   wherein the nucleic acid sequence-recognizing module is a CRISPR-Cas system wherein at least one DNA cleavage ability of Cas is inactivated.
[2] The method of [1], wherein the aforementioned Cas is deficient in two DNA cleavage abilities.
[3] The method of [1] or [2], wherein the temperature is 20°C to 25°C.
[4] The method of [1] or [2], wherein the temperature is 25°C.
[5] The method of any of [1] to [4], wherein the double-stranded DNA is contacted with the complex by introducing a nucleic acid encoding the complex into a mammalian cell having the double-stranded DNA.
[6] A method of modifying a targeted site of a double-stranded DNA, comprising a step of contacting a complex wherein a nucleic acid sequence-recognizing module that specifically binds to a target nucleotide sequence in a given double-stranded DNA, a nucleic acid base converting enzyme and a base excision repair inhibitor are bonded, with said double-stranded DNA to convert one or more nucleotides in the targeted site to other one or more nucleotides or delete one or more nucleotides, or insert one or more nucleotides into said targeted site, without cleaving at least one strand of said double-stranded DNA in the targeted site,
   wherein the nucleic acid sequence-recognizing module is a CRISPR-Cas system wherein at least one DNA cleavage ability of Cas is inactivated,
   wherein the nucleic acid base converting enzyme is PmCDA1 encoded by a nucleotide sequence having codon optimized for a host cell.
[7] The method of [6], wherein the aforementioned Cas is deficient in two DNA cleavage abilities.
[8] The method of [6] or [7], wherein the base excision repair inhibitor is a uracil DNA glycosylase inhibitor.
[9] The method according to [6], wherein the host cell is a mammalian cell.
[10] The method of any of [6] to [9], wherein the double-stranded DNA is contacted with the complex by introducing a nucleic acid encoding the complex into a cell having the double-stranded DNA.
[11] A nucleic acid-modifying enzyme complex wherein a nucleic acid sequence-recognizing module that specifically binds to a target nucleotide sequence in a given double-stranded DNA, a nucleic acid base converting enzyme and a base excision repair inhibitor are bonded, which complex converts one or more nucleotides in the targeted site to other one or more nucleotides or deletes one or more nucleotides, or inserts one or more nucleotides into said targeted site, without cleaving at least one strand of said double-stranded DNA in the targeted site,
   wherein the nucleic acid sequence-recognizing module is a CRISPR-Cas system wherein at least one DNA cleavage ability of Cas is inactivated, and
   wherein the nucleic acid base converting enzyme is PmCDA1 encoded by a nucleotide sequence having codon optimized for a host cell.
[12] The nucleic acid-modifying enzyme complex according to [11], wherein the host cell is a mammalian cell.
[13] A nucleic acid encoding the nucleic acid-modifying enzyme complex of [11] or [12].

### [Effect of the Invention]

According to the genome editing technique of the present invention, the mutation introduction efficiency is strikingly improved as compared to conventional genome editing techniques using nucleic acid base converting enzymes.

### [Brief Description of the Drawings]

Fig. 1 is a schematic showing of the genome editing plasmid used in the Examples.
Fig. 2 is a schematic showing of the evaluation method of the mutation introduction efficiency.
Fig. 3 shows the analysis results of the mutation pattern of the target gene region in the obtained mutant populations.

### [Description of Embodiments]

The present invention provides a first method of improving mutation introduction efficiency in the genome editing technique including modifying a targeted site of a double stranded DNA by converting the target nucleotide sequence and nucleotides in the vicinity thereof in the double stranded DNA to other nucleotides, without cleaving at least one chain of the double stranded DNA to be modified. The method characteristically contains a step of introducing a complex wherein a nucleic acid sequence-recognizing module that specifically binds to the target nucleotide sequence in the double-stranded DNA and PmCDA1 are bonded into a mammalian cell having the double-stranded DNA, and culturing the cell at a temperature of 20°C to 35°C at least temporarily to convert the targeted site, i.e., the target nucleotide sequence and nucleotides in the vicinity thereof, to other nucleotides, or delete the targeted site, or insert nucleotide into the site, wherein the nucleic acid sequence-recognizing module is a CRISPR-Cas system wherein at least one DNA cleavage ability of Cas is inactivated.

In one embodiment, the first method of the invention characteristically contains a step of introducing a complex wherein a nucleic acid sequence-recognizing module that specifically binds to the target nucleotide sequence in the double-stranded DNA, and a nucleic acid base converting enzyme are bonded, into a mammalian cell having the double-stranded DNA, and inhibiting base excision repair of the cell to convert the targeted site, i.e., the target nucleotide sequence and nucleotides in the vicinity thereof, to other nucleotides, or delete the targeted site, or insert nucleotide into the site.

The present invention also provides a second method of modifying a targeted site of a double-stranded DNA, comprising a step of contacting a complex wherein a nucleic acid sequence-recognizing module that specifically binds to a target nucleotide sequence in a given double-stranded DNA, a nucleic acid base converting enzyme and a base excision repair inhibitor are bonded, with said double-stranded DNA to convert one or more nucleotides in the targeted site to other one or more nucleotides or delete one or more nucleotides, or insert one or more nucleotides into said targeted site, without cleaving at least one strand of said double-stranded DNA in the targeted site,
wherein the nucleic acid sequence-recognizing module is a CRISPR-Cas system wherein at least one DNA cleavage ability of Cas is inactivated,
wherein the nucleic acid base converting enzyme is PmCDA1 encoded by a nucleotide sequence having codon optimized for a host cell.

In the present invention, the "modification" of a double-stranded DNA means that a nucleotide (e.g., dC) on a DNA strand is converted to other nucleotide (e.g., dT, dA or dG), or deleted, or a nucleotide or a nucleotide sequence is inserted between certain nucleotides on a DNA strand. While the double-stranded DNA to be modified is not particularly limited, it is preferably a genomic DNA. The "targeted site" of a double-stranded DNA means the whole or partial "target nucleotide sequence", which a nucleic acid sequence-recognizing module specifically recognizes and binds to, or the vicinity of the target nucleotide sequence (one or both of 5' upstream and 3' downstream), and the range thereof can be appropriately adjusted between 1 base and several hundred bases according to the object.

In the present invention, the "nucleic acid sequence-recognizing module" means a molecule or molecule complex having an ability to specifically recognize and bind to a particular nucleotide sequence (i.e., target nucleotide sequence) on a DNA strand. Binding of the nucleic acid sequence-recognizing module to a target nucleotide sequence enables a nucleic acid base converting enzyme and a base excision repair inhibitor linked to the module to specifically act on a targeted site of a double-stranded DNA.

In the present invention, the "nucleic acid base converting enzyme" means an enzyme capable of converting a target nucleotide to other nucleotide by catalyzing a reaction for converting a substituent on a purine or pyrimidine ring on a DNA base to other group or atom, without cleaving the DNA strand.

In the present invention, the "base excision repair" is one of the DNA repair mechanisms of living organisms, and means a mechanism for repairing damages of bases by cutting off damaged parts of the bases by enzymes and rejoining them. Excision of damaged bases is performed by DNA glycosylase, which is an enzyme that hydrolyzes the N-glycosidic bond of DNA. An abasic site (apurinic/apyrimidic (AP) site) resulting from the abasic reaction by the enzyme is treated by an enzyme at the downstream of the base excision repair (BER) pathway such as AP endonuclease, DNA polymerase, DNA ligase and the like. Examples of such gene or protein involved in the BER pathway include, but are not limited to, UNG (NM_003362), SMUG1 (NM_014311), MBD4 (NM_003925), TDG (NM_003211), OGG1 (NM_002542), MYH (NM_012222), NTHL1 (NM_002528), MPG (NM_002434), NEIL1 (NM_024608), NEIL2 (NM_145043), NEIL3 (NM_018248), APE1 (NM_001641), APE2 (NM_014481), LIG3 (NM_013975), XRCC1 (NM_006297), ADPRT (PARP1) (NM_0016718), ADPRTL2 (PARP2) (NM_005484) and the like (parentheses indicate refseq number in which the base sequence information of each gene (cDNA) is registered).

In the present invention, the "base excision repair inhibitor" means a protein that consequently inhibits BER by inhibiting any of the stages of the above-mentioned BER pathway, or inhibiting the expression itself of the molecule mobilized by the BER pathway. In the present invention, "to inhibit base excision repair" means to consequently inhibit BER by inhibiting any of the stages of the above-mentioned BER pathway, or inhibiting the expression itself of the molecule mobilized by the BER pathway.

In the present invention, the "nucleic acid-modifying enzyme complex" means a molecular complex comprising a complex comprising the above-mentioned nucleic acid sequence-recognizing module and nucleic acid base converting enzyme are connected, and having nucleic acid base converting enzyme activity and imparted with a particular nucleotide sequence recognition ability. A base excision repair inhibitor may be further linked to the complex. The "complex" here encompasses not only one constituted of multiple molecules, but also one having a nucleic acid sequence-recognizing module and a nucleic acid base converting enzyme in a single molecule, like a fusion protein. In addition, "encoding the complex" encompasses both of encoding each molecule constituting the complex and encoding a fusion protein containing the constituting molecule in a single molecule.

As disclosed herein, the "low temperature" means a temperature lower than the general culture temperature for cell proliferation in cell culture. For example, when the general culture temperature of the cell is 37°C, a temperature lower than 37°C corresponds to the low temperature. On the other hand, the low temperature needs to be a temperature that does not damage cells since the cells are damaged when the culture temperature is too low. While the low temperature varies depending on the cell type, culture period and other culture conditions, for example, when the cell is a mammalian cell such as Chinese hamster ovary (CHO) cell and the like, it is typically 20°C to 35°C, preferably 20°C to 30°C, more preferably 20°C to 25°C, further preferably 25°C.

As disclosed herein, "culturing at a low temperature at least temporarily" means culturing a cell under the above-mentioned "low temperature conditions" for at least a part of the whole culture period and encompasses culturing at a low temperature for the whole culture period. In addition, culturing cells intermittently at a low temperature in multiple times during the culture period is also encompassed in "culturing at a low temperature at least temporarily". While the timing and duration of the low temperature culture is not particularly limited, generally, low temperature culture is maintained for not less than one night after introduction of the complex of a nucleic acid sequence-recognizing module and PmCDA1 or a nucleic acid encoding same into the cell. The upper limit of the culture period is not particularly limited as long as it is the minimum period necessary for modification of the targeted site of the double-stranded DNA, and the cells may be cultured at a low temperature for the whole culture period. While the whole culture period varies depending on the cell type, culture period and other culture conditions, for example, when a mammalian cell such as CHO cell and the like is cultured at 25°C, it is typically about 10 days to 14 days. In a preferable one embodiment, a mammalian cell such as CHO cell and the like is cultured for not less than one night, preferably one night to 7 days (e.g., overnight) after introduction of the complex at 20°C to 35°C, preferably 20°C to 30°C, more preferably 20°C to 25°C, further preferably 25°C.

The nucleic acid base converting enzyme to be used in the disclosure is not particularly limited as long as it can catalyze the above-mentioned reaction, and examples thereof include deaminase belonging to the nucleic acid/nucleotide deaminase superfamily, which catalyzes a deamination reaction that converts an amino group to a carbonyl group. Preferable examples thereof include cytidine deaminase capable of converting cytosine or 5-methylcytosine to uracil or thymine, respectively, adenosine deaminase capable of converting adenine to hypoxanthine, guanosine deaminase capable of converting guanine to xanthine and the like. As cytidine deaminase, more preferred is activation-induced cytidine deaminase (hereinafter to be also referred to as AID) which is an enzyme that introduces a mutation into an immunoglobulin gene in the acquired immunity of vertebrate or the like.

While the derivation of nucleic acid base converting enzyme is not particularly limited, for example, PmCDA1 derived from Petromyzon marinus (Petromyzon marinus cytosine deaminase 1) can be used for the invention. For example, GenBank accession Nos. EF094822 and ABO15149 can be referred to for the base sequence and amino acid sequence of cDNA of PmCDA1. From the aspect of enzyme activity, PmCDA1 is preferable. As shown in the below-mentioned Examples, it was found that the risk of off-target mutation can be suppressed even when Ugi is used in combination in a particular embodiment using PmCDA1 as cytidine deaminase. Therefore, from the aspect of reduction of the risk of off-target mutation, PmCDA1 is preferable.

While the base excision repair inhibitor to be used in the disclosure is not particularly limited as long as it consequently inhibits BER, from the aspect of efficiency, an inhibitor of DNA glycosylase located at the upstream of the BER pathway is preferable. Examples of the inhibitor of DNA glycosylase to be used in the disclosure include, but are not limited to, a thymine DNA glycosylase inhibitor, an uracil DNA glycosylase inhibitor, an oxoguanine DNA glycosylase inhibitor, an alkylguanine DNA glycosylase inhibitor and the like. For example, when cytidine deaminase is used as a nucleic acid base converting enzyme, it is suitable to use a uracil DNA glycosylase inhibitor to inhibit repair of U:G or G:U mismatch of DNA generated by mutation.

Examples of such uracil DNA glycosylase inhibitor include, but are not limited to, a uracil DNA glycosylase inhibitor (Ugi) derived from Bacillus subtilis bacteriophage, PBS1, and a uracil DNA glycosylase inhibitor (Ugi) derived from Bacillus subtilis bacteriophage, PBS2 (Wang, Z., and Mosbaugh, D.W. (1988) J. Bacteriol. 170, 1082-1091). The above-mentioned inhibiter of the repair of DNA mismatch can be used in the disclosure. Particularly, Ugi derived from PBS2 is also known to have an effect of making it difficult to cause mutation, cleavage and recombination other than T from C on DNA, and thus the use of Ugi derived from PBS2 is suitable.

As mentioned above, in the base excision repair (BER) mechanism, when a base is excised by DNA glycosylase, AP endonuclease puts a nick in the abasic site (AP site), and exonuclease completely excises the AP site. When the AP site is excised, DNA polymerase produces a new base by using the base of the opposing strand as a template, and DNA ligase finally seals the nick to complete the repair. Mutant AP endonuclease that has lost the enzyme activity but maintains the binding capacity to the AP site is known to competitively inhibit BER. Therefore, these mutation AP endonucleases can also be used as the base excision repair inhibitor in the disclosure. While the derivation of the mutant AP endonuclease is not particularly limited, for example, AP endonucleases derived from Escherichia coli, yeast, mammal (e.g., human, mouse, swine, bovine, horse, monkey etc.) and the like can be used. For example, UniprotKB No. P27695 can be referred to for the amino acid sequence of human Apel. Examples of the mutant AP endonuclease that has lost the enzyme activity but maintains the binding capacity to the AP site include proteins having mutated activity site and mutated Mg (cofactor)-binding site. For example, E96Q, Y171A, Y171F, Y171H, D210N, D210A, N212A and the like can be mentioned for human Ape1.

The base excision repair of the cell can be inhibited by introducing an inhibitor of the aforementioned BER or a nucleic acid encoding same or a low-molecular-weight compound inhibiting BER. Alternatively, BER of the cell can be inhibited by suppressing the expression of a gene involved in the BER pathway. Suppression of gene expression can be performed, for example, by introducing siRNA capable of specifically suppressing expression of a gene involved in BER pathway, an antisense nucleic acid or an expression vector capable of expressing polynucleotides of these into cells. Alternatively, gene expression can be suppressed by knockout of genes involved in BER pathway.

Therefore, as one aspect of the disclosure, a method for improving the mutation introduction efficiency comprising a step of introducing a complex wherein a nucleic acid sequence-recognizing module that specifically binds to the target nucleotide sequence in the double-stranded DNA and a nucleic acid base converting enzyme are bonded into a cell containing the double-stranded DNA and showing suppressed expression of a gene relating to the BER pathway to convert the targeted site, i.e., the target nucleotide sequence and nucleotides in the vicinity thereof, to other nucleotides, or delete the targeted site, or insert nucleotide into the site is provided.

siRNA is typically a double-stranded oligo RNA consisting of an RNA having a sequence complementary to a nucleotide sequence of mRNA or a partial sequence thereof (hereinafter target nucleotide sequence) of the target gene, and a complementary strand thereof. The nucleotide sequences of these RNAs can be appropriately designed according to the sequence information of the genes involved in the BER pathway. It is a single-stranded RNA in which a sequence complementary to the target nucleotide sequence (first sequence) and a sequence complementary thereto (second sequence) are linked via a hairpin loop portion, and an RNA (small hairpin RNA: shRNA) in which the first sequence forms a double-stranded structure with the second sequence by adopting a hairpin loop type structure is also one of the preferable aspects of siRNA.

The antisense nucleic acid means a nucleic acid containing a nucleotide sequence capable of specifically hybridizing with the target mRNA under physiological conditions of cells expressing the target mRNA (mature mRNA or initial transcription product) and capable of inhibiting translation of polypeptide coded by the target mRNA while being hybridized. The kind of the antisense nucleic acid may be DNA or RNA, or DNA/RNA chimera. The nucleotide sequence of these nucleic acids can be appropriately designed according to the sequence information of a gene involved in the BER pathway.

Knockout of genes involved in the BER pathway means that all or a part of the genes involved in the BER pathway have been destroyed or recombined so as not to exhibit their original functions. The gene may be destroyed or mutated so that one allele on the genome will not function and plural alleles may be destroyed or mutated. Knockout can be performed by a known method. For example, a method of knocking out by introducing a DNA construct made to cause genetic recombination with the target gene into the cell, a method of knocking out by insertion, deletion, substitution introduction of bases by using TALEN, CRISPR-Cas9 system or the like can be mentioned.

A target nucleotide sequence in a double-stranded DNA to be recognized by the nucleic acid sequence-recognizing module in the nucleic acid-modifying enzyme complex of the present invention is not particularly limited as long as the module specifically binds to, and may be any sequence in the double-stranded DNA. The length of the target nucleotide sequence only needs to be sufficient for specific binding of the nucleic acid sequence-recognizing module. For example, when mutation is introduced into a particular site in the genomic DNA of a mammal, it is not less than 12 nucleotides, preferably not less than 15 nucleotides, more preferably not less than 17 nucleotides, according to the genome size thereof. While the upper limit of the length is not particularly limited, it is preferably not more than 25 nucleotides, more preferably not more than 22 nucleotides.

As the nucleic acid sequence-recognizing module in the nucleic acid-modifying enzyme complex of the present invention, CRISPR-Cas system wherein at least one DNA cleavage ability of Cas is inactivated (hereinafter to be also referred to as "CRISPR-mutant Cas" and also encompasses CRISPR-mutant Cpf1), zinc finger motif, TAL effector and PPR motif and the like, as well as a fragment containing a DNA binding domain of a protein that specifically binds to DNA, such as restriction enzyme, transcription factor, RNA polymerase and the like, and free of a DNA double strand cleavage ability and the like can be used, but the module is not limited thereto. Preferably, CRISPR-mutant Cas, zinc finger motif, TAL effector, PPR motif and the like can be mentioned.

As a genome editing technique using CRISPR, a case using CRISPR-Cpfl has been reported besides CRISPR-Cas9 (Zetsche B., et al., Cell, 163:759-771 (2015)). Cpf1 has properties different from Cas9 in that it does not require tracrRNA, that the cleaved DNA is a cohesive end, that the PAM sequence is present on the 5'-side and is a T-rich sequence and the like. Cpf1 capable of genome editing in mammalian cells includes, but is not limited to, Cpf1 derived from Acidaminococcus sp. BV3L6, Cpf1 derived from Lachnospiraceae bacterium ND2006 and the like. Mutant Cpf1 lacking DNA cleavage ability includes a D917A mutant in which the 917th Asp residue of Cpf1 (FnCpf1) derived from Francisella novicida U112 is converted to an Ala residue, an E1006A mutant obtained by converting the 1006th Glu residue to an Ala residue, a D1255A mutant obtained by converting the 1255th Asp residue to an Ala residue and the like. The mutant is not limited to these mutants and any mutant Cpf1 lacking the DNA cleavage ability can be used in the present invention.

A zinc finger motif is constituted by linkage of 3 - 6 different Cys2His2 type zinc finger units (1 finger recognizes about 3 bases), and can recognize a target nucleotide sequence of 9 - 18 bases. A zinc finger motif can be produced by a known method such as Modular assembly method (Nat Biotechnol (2002) 20: 135-141), OPEN method (Mol Cell (2008) 31: 294-301), CoDA method (Nat Methods (2011) 8: 67-69), Escherichia coli one-hybrid method (Nat Biotechnol (2008) 26:695-701) and the like. The above-mentioned patent document 1 can be referred to as for the detail of the zinc finger motif production.

A TAL effector has a module repeat structure with about 34 amino acids as a unit, and the 12th and 13th amino acid residues (called RVD) of one module determine the binding stability and base specificity. Since each module is highly independent, TAL effector specific to a target nucleotide sequence can be produced by simply connecting the module. For TAL effector, a production method utilizing an open resource (REAL method (Curr Protoc Mol Biol (2012) Chapter 12: Unit 12.15), FLASH method (Nat Biotechnol (2012) 30: 460-465), and Golden Gate method (Nucleic Acids Res (2011) 39: e82) etc.) have been established, and a TAL effector for a target nucleotide sequence can be designed comparatively conveniently. The above-mentioned patent document 2 can be referred to as for the detail of the production of TAL effector.

PPR motif is constituted such that a particular nucleotide sequence is recognized by a continuation of PPR motifs each consisting of 35 amino acids and recognizing one nucleic acid base, and recognizes a target base only by 1, 4 and ii(-2) amino acids of each motif. Motif constituent has no dependency, and is free of interference of motifs on both sides. Therefore, like TAL effector, a PPR protein specific to the target nucleotide sequence can be produced by simply connecting PPR motifs. The above-mentioned patent document 4 can be referred to as for the detail of the production of PPR motif.

When a fragment of restriction enzyme, transcription factor, RNA polymerase and the like is used, since the DNA binding domains of these proteins are well known, a fragment containing the domain and free of a DNA double strand cleavage ability can be easily designed and constructed.

Any of the above-mentioned nucleic acid sequence-recognizing module can be provided as a fusion protein with the above-mentioned nucleic acid base converting enzyme and/or base excision repair inhibitor, or a protein binding domain such as SH3 domain, PDZ domain, GK domain, GB domain and the like and a binding partner thereof may be fused with a nucleic acid sequence-recognizing module and a nucleic acid base converting enzyme and/or base excision repair inhibitor, respectively, and provided as a protein complex via an interaction of the domain and a binding partner thereof. Alternatively, a nucleic acid sequence-recognizing module and a nucleic acid base converting enzyme and/or base excision repair inhibitor may be each fused with intein, and they can be linked by ligation after protein synthesis.

The nucleic acid-modifying enzyme complex of the present invention may be contacted with a double-stranded DNA by introducing the complex or a nucleic acid encoding the complex into a cell having the object double-stranded DNA (e.g., genomic DNA). In consideration of the introduction and expression efficiency, it is desirable to introduce the complex in the form of a nucleic acid encoding same rather than the nucleic acid modifying enzyme complex itself, and express the complex in the cell.

Therefore, the nucleic acid sequence-recognizing module, the nucleic acid base converting enzyme and the base excision repair inhibitor are preferably prepared as a nucleic acid encoding a fusion protein thereof, or in a form capable of forming a complex in a host cell after translation into a protein by utilizing a binding domain, intein and the like, or as a nucleic acid encoding each of them. The nucleic acid here may be a DNA or an RNA. When it is a DNA, it is preferably a double-stranded DNA, and provided in the form of an expression vector disposed under regulation of a functional promoter in a host cell. When it is an RNA, it is preferably a single-stranded RNA.

Since the complex of the present invention does not accompany double-stranded DNA breaks (DSB), genome editing with low toxicity is possible, and the genetic modification method of the present invention can be applied to a wide range of biological materials. Therefore, the cells to be introduced with nucleic acid encoding the above-mentioned nucleic acid converting enzyme complex can encompass cells of any species, from bacterium of Escherichia coli and the like which are prokaryotes, cells of microorganism such as yeast and the like which are lower eucaryotes, to cells of vertebrate including mammals such as human and the like, and cells of higher eukaryote such as insect, plant and the like.

A DNA encoding a nucleic acid sequence-recognizing module such as zinc finger motif, TAL effector, PPR motif and the like can be obtained by any method mentioned above for each module. A DNA encoding a sequence-recognizing module of restriction enzyme, transcription factor, RNA polymerase and the like can be cloned by, for example, synthesizing an oligoDNA primer covering a region encoding a desired part of the protein (part containing DNA binding domain) based on the cDNA sequence information thereof, and amplifying by the RT-PCR method using, as a template, the total RNA or mRNA fraction prepared from the protein-producing cells.

A DNA encoding a nucleic acid base converting enzyme and base excision repair inhibitor can also be cloned similarly by synthesizing an oligoDNA primer based on the cDNA sequence information thereof, and amplifying by the RT-PCR method using, as a template, the total RNA or mRNA fraction prepared from the enzyme-producing cells. For example, a DNA encoding PBS2-derived Ugi can be cloned by designing suitable primers for the upstream and downstream of CDS based on the DNA sequence (accession No. J04434) registered in the NCBI/GenBank database, and cloning from PBS2-derived mRNA by the RT-PCR method.

The cloned DNA may be directly used as a DNA encoding a protein, or prepared into a DNA encoding a protein after digestion with a restriction enzyme when desired, or after addition of a suitable linker (e.g., GS linker, GGGAR linker etc.), spacer (e.g., FLAG sequence etc.) and/or a nuclear localization signal (NLS) (each organelle transfer signal when the object double-stranded DNA is mitochondria or chloroplast DNA). It may be further ligated with a DNA encoding a nucleic acid sequence-recognizing module to prepare a DNA encoding a fusion protein.

Alternatively, a DNA encoding a nucleic acid modification enzyme complex may be fused with a DNA encoding a binding domain or a binding partner thereof, or both DNAs may be fused with a DNA encoding a separation intein, whereby the nucleic acid sequence-recognizing conversion module and the nucleic acid modification enzyme complex are translated in a host cell to form a complex. In these cases, a linker and/or a nuclear localization signal can be linked to a suitable position of one of or both DNAs when desired.

A DNA encoding a nucleic acid modification enzyme complex can be obtained by chemically synthesizing the DNA strand, or by connecting synthesized partly overlapping oligoDNA short strands by utilizing the PCR method and the Gibson Assembly method to construct a DNA encoding the full length thereof. The advantage of constructing a full-length DNA by chemical synthesis or a combination of PCR method or Gibson Assembly method is that the codon to be used can be designed in CDS full-length according to the host into which the DNA is introduced. In the expression of a heterologous DNA, the protein expression level is expected to increase by converting the DNA sequence thereof to a codon highly frequently used in the host organism. As the data of codon use frequency in host to be used, for example, the genetic code use frequency database (http://www.kazusa.or.jp/codon/index.html) disclosed in the home page of Kazusa DNA Research Institute can be used, or documents showing the codon use frequency in each host may be referred to. By reference to the obtained data and the DNA sequence to be introduced, codons showing low use frequency in the host from among those used for the DNA sequence may be converted to a codon coding the same amino acid and showing high use frequency.

An expression vector containing a DNA encoding a nucleic acid modification enzyme complex can be produced, for example, by linking the DNA to the downstream of a promoter in a suitable expression vector.

As the expression vector, Escherichia coli-derived plasmids (e.g., pBR322, pBR325, pUC12, pUC13); Bacillus subtilis-derived plasmids (e.g., pUB110, pTP5, pC194); yeast-derived plasmids (e.g., pSH19, pSH15); insect cell expression plasmids (e.g., pFast-Bac); animal cell expression plasmids (e.g., pA1-11, pXT1, pRc/CMV, pRc/RSV, pcDNAI/Neo); bacteriophages such as λphage and the like; insect virus vectors such as baculovirus and the like (e.g., BmNPV, AcNPV); animal virus vectors such as retrovirus, vaccinia virus, adenovirus and the like, and the like are used.

As the promoter, any promoter appropriate for a host to be used for gene expression can be used. In a conventional method accompanying DSB, since the survival rate of the host cell sometimes decreases markedly due to the toxicity, it is desirable to increase the number of cells by the start of the induction by using an inductive promoter. However, since sufficient cell proliferation can also be afforded by expressing the nucleic acid-modifying enzyme complex of the present invention, a constituent promoter can also be used without limitation.

For example, when the host is an animal cell, SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (simple herpes virus thymidine kinase) promoter and the like are used. Of these, CMV promoter, SRα promoter and the like are preferable.

When the host is Escherichia coli, trp promoter, lac promoter, recA promoter, λP_{L} promoter, lpp promoter, T7 promoter and the like are preferable.

When the host is genus Bacillus, SPO1 promoter, SPO2 promoter, penP promoter and the like are preferable.

When the host is a yeast, Gal1/10 promoter, PHO5 promoter, PGK promoter, GAP promoter, ADH promoter and the like are preferable.

When the host is an insect cell, polyhedrin promoter, P10 promoter and the like are preferable.

When the host is a plant cell, CaMV35S promoter, CaMV19S promoter, NOS promoter and the like are preferable.

As the expression vector, besides those mentioned above, one containing enhancer, splicing signal, terminator, polyA addition signal, a selection marker such as drug resistance gene, auxotrophic complementary gene and the like, replication origin and the like on demand can be used.

An RNA encoding a nucleic acid modification enzyme complex can be prepared by, for example, transcription to mRNA in a vitro transcription system known per se by using a vector containing a DNA encoding each protein as a template.

The complex of the present invention can be intracellularly expressed by introducing an expression vector containing a DNA encoding a nucleic acid modification enzyme complex, and culturing the host cell.

As the host, genus Escherichia, genus Bacillus, yeast, insect cell, insect, animal cell and the like are used.

As the genus Escherichia, Escherichia coli K12.DH1 [Proc. Natl. Acad. Sci. USA, 60, 160 (1968)], Escherichia coli JM103 [Nucleic Acids Research, 9, 309 (1981)], Escherichia coli JA221 [Journal of Molecular Biology, 120, 517 (1978)], Escherichia coli HB101 [Journal of Molecular Biology, 41, 459 (1969)], Escherichia coli C600 [Genetics, 39, 440 (1954)] and the like are used.

As the genus Bacillus, Bacillus subtilis MI114 [Gene, 24, 255 (1983)], Bacillus subtilis 207-21 [Journal of Biochemistry, 95, 87 (1984)] and the like are used.

As the yeast, Saccharomyces cerevisiae AH22, AH22R⁻, NA87-11A, DKD-5D, 20B-12, Schizosaccharomyces pombe NCYC1913, NCYC2036, Pichia pastoris KM71 and the like are used.

As the insect cell when the virus is AcNPV, cells of cabbage armyworm larva-derived established line (Spodoptera frugiperda cell; Sf cell), MG1 cells derived from the mid-intestine of Trichoplusia ni, High Five^{™} cells derived from an egg of Trichoplusia ni, Mamestra brassicae-derived cells, Estigmena acrea-derived cells and the like are used. When the virus is BmNPV, cells of Bombyx mori-derived established line (Bombyx mori N cell; BmN cell) and the like are used as insect cells. As the Sf cell, for example, Sf9 cell (ATCC CRL1711), Sf21 cell [all above, In Vivo, 13, 213-217 (1977)] and the like are used.

As the insect, for example, larva of Bombyx mori, Drosophila, cricket and the like are used [Nature, 315, 592 (1985)].

As the animal cell, cell lines such as monkey COS-7 cell, monkey Vero cell, CHO cell, dhfr gene-deficient CHO cell, mouse L cell, mouse AtT-20 cell, mouse myeloma cell, rat GH3 cell, human FL cell, human fetal kidney-derived cells (e.g., HEK293 cell) and the like, pluripotent stem cells such as iPS cell, ES cell and the like of human and other mammals, and primary cultured cells prepared from various tissues are used. Furthermore, zebrafish embryo, Xenopus oocyte and the like can also be used.

As the plant cell, suspend cultured cells, callus, protoplast, leaf segment, root segment and the like prepared from various plants (e.g., grain such as rice, wheat, corn and the like, product crops such as tomato, cucumber, egg plant and the like, garden plants such as carnation, Eustoma russellianum and the like, experiment plants such as tobacco, arabidopsis thaliana and the like, and the like) are used.

All the above-mentioned host cells may be haploid (monoploid), or polyploid (e.g., diploid, triploid, tetraploid and the like).

An expression vector can be introduced by a known method (e.g., lysozyme method, competent method, PEG method, CaCl₂ coprecipitation method, electroporation method, the microinjection method, the particle gun method, lipofection method, Agrobacterium method and the like) according to the kind of the host.

Escherichia coli can be transformed according to the methods described in, for example, Proc. Natl. Acad. Sci. USA, 69, 2110 (1972), Gene, 17, 107 (1982) and the like.

The genus Bacillus can be introduced into a vector according to the methods described in, for example, Molecular & General Genetics, 168, 111 (1979) and the like.

A yeast can be introduced into a vector according to the methods described in, for example, Methods in Enzymology, 194, 182-187 (1991), Proc. Natl. Acad. Sci. USA, 75, 1929 (1978) and the like.

An insect cell and an insect can be introduced into a vector according to the methods described in, for example, Bio/Technology, 6, 47-55 (1988) and the like.

An animal cell can be introduced into a vector according to the methods described in, for example, Cell Engineering additional volume 8, New Cell Engineering Experiment Protocol, 263-267 (1995) (published by Shujunsha), and Virology, 52, 456 (1973).

A cell introduced with a vector can be cultured according to a known method according to the kind of the host.

For example, when Escherichia coli or genus Bacillus is cultured, a liquid medium is preferable as a medium to be used for the culture. The medium preferably contains a carbon source, nitrogen source, inorganic substance and the like necessary for the growth of the transformant. Examples of the carbon source include glucose, dextrin, soluble starch, sucrose and the like; examples of the nitrogen source include inorganic or organic substances such as ammonium salts, nitrate salts, corn steep liquor, peptone, casein, meat extract, soybean cake, potato extract and the like; and examples of the inorganic substance include calcium chloride, sodium dihydrogen phosphate, magnesium chloride and the like. The medium may contain yeast extract, vitamins, growth promoting factor and the like. The pH of the medium is preferably about 5 - about 8.

As a medium for culturing Escherichia coli, for example, M9 medium containing glucose, casamino acid [Journal of Experiments in Molecular Genetics, 431-433, Cold Spring Harbor Laboratory, New York 1972] is preferable. Where necessary, for example, agents such as 3β-indolylacrylic acid may be added to the medium to ensure an efficient function of a promoter. Escherichia coli is cultured at generally about 15 - about 43°C. Where necessary, aeration and stirring may be performed.

The genus Bacillus is cultured at generally about 30 - about 40°C. Where necessary, aeration and stirring may be performed.

Examples of the medium for culturing yeast include Burkholder minimum medium [Proc. Natl. Acad. Sci. USA, 77, 4505 (1980)], SD medium containing 0.5% casamino acid [Proc. Natl. Acad. Sci. USA, 81, 5330 (1984)] and the like. The pH of the medium is preferably about 5 - about 8. The culture is performed at generally about 20°C - about 35°C. Where necessary, aeration and stirring may be performed.

As a medium for culturing an insect cell or insect, for example, Grace's Insect Medium [Nature, 195, 788 (1962)] containing an additive such as inactivated 10% bovine serum and the like as appropriate and the like are used. The pH of the medium is preferably about 6.2 - about 6.4. The culture is performed at generally about 27°C. Where necessary, aeration and stirring may be performed.

As a medium for culturing an animal cell, for example, minimum essential medium (MEM) containing about 5 - about 20% of fetal bovine serum [Science, 122, 501 (1952)], Ham's F12 medium, Dulbecco's modified Eagle medium (DMEM) [Virology, 8, 396 (1959)], RPMI 1640 medium [The Journal of the American Medical Association, 199, 519 (1967)], 199 medium [Proceeding of the Society for the Biological Medicine, 73, 1 (1950)] and the like are used. The pH of the medium is preferably about 6 - about 8. The culture is performed at generally about 30°C - about 40°C. Where necessary, aeration and stirring may be performed.

As a medium for culturing a plant cell, for example, MS medium, LS medium, B5 medium and the like are used. The pH of the medium is preferably about 5 - about 8. The culture is performed at generally about 20°C - about 30°C. Where necessary, aeration and stirring may be performed.

The culture period is not particularly limited as long as it is at least the period necessary for the targeted site of the double-stranded DNA to be modified, and can be appropriately selected according to the host cell. To avoid undesirable off-target mutation, preferably, the culture is not performed beyond a period sufficient to modify the targeted site. The timing and duration of the low temperature culture when performing the step of culturing at a low temperature at least temporarily is as described above.

As mentioned above, nucleic acid modification enzyme complex can be expressed intracellularly.

An RNA encoding a nucleic acid modification enzyme complex can be introduced into a host cell by microinjection method, lipofection method and the like. RNA introduction can be performed once or repeated multiple times (e.g., 2 - 5 times) at suitable intervals.

When a complex of a nucleic acid sequence-recognizing module and a nucleic acid base converting enzyme is expressed by an expression vector or RNA molecule introduced into the cell, the nucleic acid sequence-recognizing module specifically recognizes and binds to a target nucleotide sequence in the double-stranded DNA (e.g., genomic DNA) of interest and, due to the action of the nucleic acid base converting enzyme linked to the nucleic acid sequence-recognizing module, base conversion occurs in the sense strand or antisense strand of the targeted site (whole or partial target nucleotide sequence or appropriately adjusted within several hundred bases including the vicinity thereof) and a mismatch occurs in the double-stranded DNA (e.g., when cytidine deaminase such as PmCDA1, AID and the like is used as a nucleic acid base converting enzyme, cytosine on the sense strand or antisense strand at the targeted site is converted to uracil to cause U:G or G:U mismatch). When the mismatch is not correctly repaired, and when repaired such that a base of the opposite strand forms a pair with a base of the converted strand (T-A or A-T in the above-mentioned example), or when other nucleotide is further substituted (e.g., U→A, G) or when one to several dozen bases are deleted or inserted during repair, various mutations are introduced. By using inhibitors of base excision repair in combination, the BER mechanism in cells is inhibited, the frequency of repair error is increased, and mutation introduction efficiency can be improved.

As for zinc finger motif, production of many actually functionable zinc finger motifs is not easy, since production efficiency of a zinc finger that specifically binds to a target nucleotide sequence is not high and selection of a zinc finger having high binding specificity is complicated. While TAL effector and PPR motif have a high degree of freedom of target nucleic acid sequence recognition as compared to zinc finger motif, a problem remains in the efficiency since a large protein needs to be designed and constructed every time according to the target nucleotide sequence.

In contrast, since the CRISPR-Cas system recognizes the object double-stranded DNA sequence by a guide RNA complementary to the target nucleotide sequence, any sequence can be targeted by simply synthesizing an oligoDNA capable of specifically forming a hybrid with the target nucleotide sequence.

Therefore, in a more preferable embodiment of the present invention, a CRISPR-Cas system wherein at least one DNA cleavage ability of Cas is inactivated (CRISPR-mutant Cas) is used as a nucleic acid sequence-recognizing module.

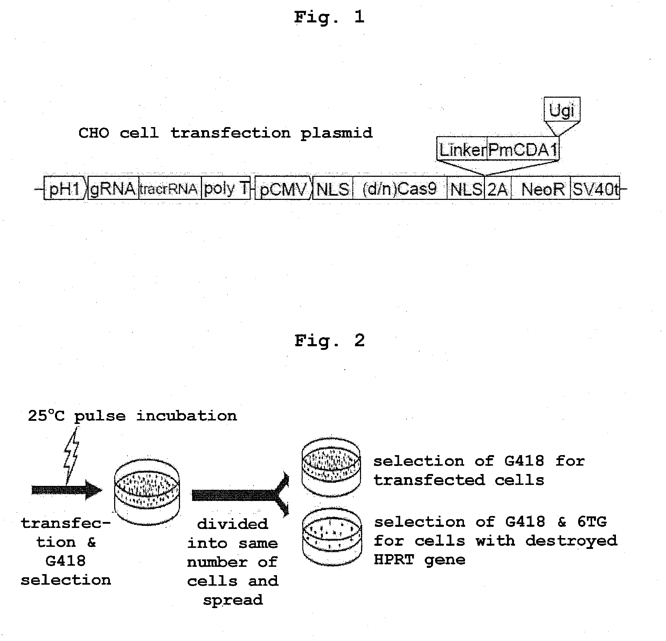

Fig. 1 is a schematic showing of the genome editing plasmid of the present invention using CRISPR-mutant Cas as a nucleic acid sequence-recognizing module.

The nucleic acid sequence-recognizing module of the present invention using CRISPR-mutant Cas is provided as a complex of an RNA molecule consisting of a guide RNA (gRNA) complementary to the target nucleotide sequence and tracrRNA necessary for recruiting mutant Cas protein, and a mutant Cas protein.

The Cas protein to be used in the present invention is not particularly limited as long as it belongs to the CRISPR system, and preferred is Cas9. Examples of Cas9 include, but are not limited to, Streptococcus pyogenes-derived Cas9 (SpCas9), Streptococcus thermophilus-derived Cas9 (StCas9) and the like. Preferred is SpCas9. As a mutant Cas to be used in the present invention, any of Cas wherein the cleavage ability of the both strands of the double-stranded DNA is inactivated and one having nickase activity wherein at least one cleavage ability of one strand alone is inactivated can be used. For example, in the case of SpCas9, a D10A mutant wherein the 10th Asp residue is converted to an Ala residue and lacking cleavage ability of a strand opposite to the strand forming a complementary strand with a guide RNA, or H840A mutant wherein the 840th His residue is converted to an Ala residue and lacking cleavage ability of strand complementary to guide RNA, or a double mutant thereof can be used, and other mutant Cas can be used similarly.

A nucleic acid base converting enzyme and base excision repair inhibitor are provided as a complex with mutant Cas by a method similar to the coupling scheme with the above-mentioned zinc finger and the like. Alternatively, a nucleic acid base converting enzyme and/or base excision repair inhibitor and mutant Cas can also be bound by utilizing RNA aptamers MS2F6, PP7 and the like and RNA scaffold by binding proteins thereto. Guide RNA forms a complementary strand with the target nucleotide sequence, mutant Cas is recruited by the tracrRNA attached and mutant Cas recognizes DNA cleavage site recognition sequence PAM (protospacer adjacent motif) (when SpCas9 is used, PAM is 3 bases of NGG (N is any base), and, theoretically, can target any position on the genome). One or both DNAs cannot be cleaved, and, due to the action of the nucleic acid base converting enzyme linked to the mutant Cas, nucleic acid base conversion occurs in the targeted site (appropriately adjusted within several hundred bases including whole or partial target nucleotide sequence) and a mismatch occurs in the double-stranded DNA. Due to the error of the BER system of the cell to be repaired, various mutations are introduced.

When CRISPR-mutant Cas is used as a nucleic acid sequence-recognizing module, CRISPR-mutant Cas is desirably introduced, in the form of a nucleic acid encoding nucleic acid modification enzyme complex, into a cell having a double-stranded DNA of interest, similar to when zinc finger and the like are used as a nucleic acid sequence-recognizing module.

A DNA encoding Cas can be cloned by a method similar to the above-mentioned method for a DNA encoding a base excision repair inhibitor, from a cell producing the enzyme. A mutant Cas can be obtained by introducing a mutation to convert an amino acid residue of the part important for the DNA cleavage activity (e.g., 10th Asp residue and 840th His residue for Cas9, though not limited thereto) to other amino acid, into a DNA encoding cloned Cas, by a site specific mutation induction method known per se.

Alternatively, a DNA encoding mutant Cas can also be constructed as a DNA having codon usage suitable for expression in a host cell to be used, by a method similar to those mentioned above for a DNA encoding a nucleic acid sequence-recognizing module and a DNA encoding a DNA glycosylase, and by a combination of chemical synthesis or PCR method or Gibson Assembly method. For example, CDS sequence and amino acid sequence optimized for the expression of SpCas9 in eukaryotic cells are shown in SEQ ID NOs: 3 and 4. In the sequence shown in SEQ ID NO: 3, when "A" is converted to "C" in base No. 29, a DNA encoding a D10A mutant can be obtained, and when "CA" is converted to "GC" in base No. 2518-2519, a DNA encoding an H840A mutant can be obtained.

A DNA encoding a mutant Cas and a DNA encoding a nucleic acid base converting enzyme may be linked to allow for expression as a fusion protein, or designed to be separately expressed using a binding domain, intein and the like, and form a complex in a host cell via protein-protein interaction or protein ligation. Alternatively, a design may be employed in which a DNA encoding mutant Cas and a DNA encoding a nucleic acid base converting enzyme are each split into two fragments at suitable split site, either fragments are linked to each other directly or via a suitable linker to express a nucleic acid-modifying enzyme complex as two partial complexes, which are associated and refolded in the cell to reconstitute functional mutant Cas having a particular nucleic acid sequence recognition ability, and a functional nucleic acid base converting enzyme having a nucleic acid base conversion reaction catalyst activity is reconstituted when the mutant Cas is bonded to the target nucleotide sequence. For example, a DNA encoding the N-terminal side fragment of mutant Cas9 and a DNA encoding the C-terminal side fragment of mutant Cas are respectively prepared by the PCR method by using suitable primers; a DNA encoding the N-terminal side fragment of a nucleic acid base converting enzyme and a DNA encoding the C-terminal side fragment of a nucleic acid base converting enzyme are prepared in the same manner; for example, the DNAs encoding the N-terminal side fragments are linked to each other, and the DNAs encoding the C-terminal side fragments are linked to each other by a conventional method, whereby a DNA encoding two partial complexes can be produced. Alternatively, a DNA encoding the N-terminal side fragment of mutant Cas and a DNA encoding the C-terminal side fragment of a nucleic acid base converting enzyme are linked; and a DNA encoding the N-terminal side fragment of a nucleic acid base converting enzyme and a DNA encoding the C-terminal side fragment of mutant Cas are linked, whereby a DNA encoding two partial complexes can also be produced. Respective partial complexes may be linked to allow for expression as a fusion protein, or designed to be separately expressed using a binding domain, intein and the like, and form a complex in a host cell via protein-protein interaction or protein ligation. Two partial complexes may be linked to be expressed as a fusion protein. The split site of the mutant Cas is not particularly limited as long as the two split fragments can be reconstituted such that they recognize and bind to the target nucleotide sequence, and it may be split at one site to provide N-terminal side fragment and C-terminal side fragment, or not less than 3 fragments obtained by splitting at two or more sites may be appropriately linked to give two fragments. The three-dimensional structures of various Cas proteins are known, and those of ordinary skill in the art can appropriately select the split site based on such information. For example, since the region consisting of the 94th to the 718th amino acids from the N terminus of SpCas9 is a domain (REC) involved in the recognition of the target nucleotide sequence and guide RNA, and the region consisting of the 1099th amino acid to the C-terminal amino acid is the domain (PI) involved in the interaction with PAM, the N-terminal side fragment and the C-terminal side fragment can be split at any site in REC domain or PI domain, preferably in a region free of a structure (e.g., between 204th and 205th amino acid from the N-terminal (204..205), between 535th and 536th amino acids from the N-terminal (535..536) and the like) (see, for example, Nat Biotechnol. 33(2): 139-142 (2015)). A combination of a DNA encoding a base excision repair inhibitor and a DNA encoding a mutant Cas and/or a DNA encoding a nucleic acid base converting enzyme can also be designed in the same manner as described above.

The obtained DNA encoding a mutant Cas and/or a nucleic acid base converting enzyme and/or base excision repair inhibitor can be inserted into the downstream of a promoter of an expression vector similar to the one mentioned above, according to the host.

On the other hand, a DNA encoding guide RNA and tracrRNA can be obtained by designing an oligoDNA sequence linking a coding sequence of crRNA sequence containing a nucleotide sequence complementary to the target nucleotide sequence (e.g., when FnCpf1 is recruited as Cas, crRNA containing SEQ ID NO: 20; AAUUUCUACUGUUGUAGAU at the 5'-side of the complementary nucleotide sequence can be used, and underlined sequences form base pairs to take a stem-loop structure), or a crRNA coding sequence and, as necessary, a known tracrRNA coding sequence (e.g., as tracrRNA coding sequence when Cas9 is recruited as Cas, gttttagagctagaaatagcaagttaaaataaggctagtccgttatcaacttgaaaaagtggc accgagtcggtggtgctttt; SEQ ID NO: 9) and chemically synthesizing using a DNA/RNA synthesizer. While a DNA encoding guide RNA and tracrRNA can also be inserted into an expression vector similar to the one mentioned above, according to the host. As the promoter, pol III system promoter (e.g., SNR6, SNR52, SCR1, RPR1, U6, H1 promoter etc.) and terminator (e.g., T₆ sequence) are preferably used.

An RNA encoding mutant Cas and/or a nucleic acid base converting enzyme and/or base excision repair inhibitor can be prepared by, for example, transcription to mRNA in vitro transcription system known per se by using a vector encoding the above-mentioned mutant Cas and/or a nucleic acid base converting enzyme and/or base excision repair inhibitor as a template.

Guide RNA-tracrRNA can be obtained by designing an oligoDNA sequence linking a sequence complementary to the target nucleotide sequence and known tracrRNA sequence and chemically synthesizing using a DNA/RNA synthesizer.

A DNA or RNA encoding mutant Cas and/or a nucleic acid base converting enzyme and/or base excision repair inhibitor, guide RNA-tracrRNA or a DNA encoding same can be introduced into a host cell by a method similar to the above, according to the host.

Since conventional artificial nuclease accompanies Double-stranded DNA breaks (DSB), inhibition of growth and cell death assumedly caused by disordered cleavage of chromosome (off-target cleavage) occur by targeting a sequence in the genome. The effect thereof is particularly fatal for many microorganisms and prokaryotes, and prevents applicability. In the method of the present invention, cytotoxicity is drastically reduced as compared to a method using a conventional artificial nuclease since mutation introduction is performed not by DNA cleavage but by nucleic acid base conversion reaction on DNA.

When sequence-recognizing modules are produced corresponding to the adjacent multiple target nucleotide sequences, and simultaneously used, the mutation introduction efficiency can increase more than using a single nucleotide sequence as a target. As the effect thereof, similarly mutation induction is realized even when both target nucleotide sequences partly overlap or when the both are apart by about 600 bp. It can occur both when the target nucleotide sequences are in the same direction (target nucleotide sequences are present on the same strand), and when they are opposed (target nucleotide sequence is present on each strand of double-stranded DNA).

Since the genome editing technique of the present invention shows extremely high mutation introduction efficiency, modification of multiple DNA regions at completely different positions as targets can be performed. Therefore, in one preferable embodiment of the present invention, two or more kinds of nucleic acid sequence-recognizing modules that specifically bind to different target nucleotide sequences (which may be present in one object gene, or two or more different object genes, which object genes may be present on the same chromosome or different chromosomes) can be used. In this case, each one of these nucleic acid sequence-recognizing modules, a nucleic acid base converting enzyme and a base excision repair inhibitor form a nucleic acid-modifying enzyme complex. Here, a common nucleic acid base converting enzyme and a base excision repair inhibitor can be used. For example, when CRISPR-Cas system is used as a nucleic acid sequence-recognizing module, a common complex of a Cas protein, a nucleic acid base converting enzyme and a base excision repair inhibitor (including fusion protein) are used, and two or more kinds of chimeric RNAs of tracrRNA and each of two or more guide RNAs that respectively form a complementary strand with a different target nucleotide sequence are produced and used as guide RNA-tracrRNAs. On the other hand, when zinc finger motif, TAL effector and the like are used as nucleic acid sequence-recognizing modules, for example, a nucleic acid base converting enzyme and base excision repair inhibitor can be fused with a nucleic acid sequence-recognizing module that specifically binds to a different target nucleotide.

To express the nucleic acid-modifying enzyme complex of the present invention in a host cell, as mentioned above, an expression vector containing a DNA encoding the nucleic acid-modifying enzyme complex, or an RNA encoding the nucleic acid-modifying enzyme complex is introduced into a host cell. For efficient introduction of mutation, it is desirable to maintain an expression of nucleic acid-modifying enzyme complex of a given level or above for not less than a given period. From such aspect, it is ensuring to introduce an expression vector (plasmid etc.) autonomously replicatable in a host cell. However, since the plasmid etc. are foreign DNAs, they are preferably removed rapidly after successful introduction of mutation. Therefore, though subject to change depending on the kind of host cell and the like, for example, the introduced plasmid is desirably removed from the host cell after a lapse of 6 hr - 2 days from the introduction of an expression vector by using various plasmid removal methods well known in the art.

Alternatively, as long as expression of a nucleic acid-modifying enzyme complex, which is sufficient for the introduction of mutation, is obtained, it is preferable to introduce mutation into the object double-stranded DNA by transient expression by using an expression vector or RNA without autonomous replicatability in a host cell (e.g., vector etc. lacking replication origin that functions in host cell and/or gene encoding protein necessary for replication).

The present invention is explained in the following by referring to Examples, which are not to be construed as limitative.

### [Examples]

In the below-mentioned Examples, experiments were performed as follows.

### <Cell line•culture•transformation•expression induction>

CHO-K1 adherent cell derived from Chinese hamster ovary was used. The cell was cultured in a hamF12 medium (Life Technologies, Carlsbad, CA, USA) supplemented with 10% fetal bovine serum (Biosera, Nuaille, France) and 100 µg/mL penicillin-streptomycin (Life Technologies, Carlsbad, CA, USA). The cells were incubated under humidified 5% CO₂ atmosphere at 37°C. For transfection, a 24 well plate was used and the cells were seeded at 0.5×10⁵ cells per well and cultured for one day. According to the manufacturer's instructions, 1.5 µg of plasmid and 2 µL of lipofectamine 2000 (Life Technologies, Carlsbad, CA, USA) were transfected into the cells. After 5 hr from the transfection, the medium was exchanged with hamF12 medium containing 0.125 mg/mL G418 (InvivoGen, San Diego, CA, USA) and the cells were incubated for 7 days. Thereafter, the cells were used for the calculation of the following mutation introduction efficiency.

The step of culturing at a low temperature temporarily included transfection similar to the above-mentioned, medium exchange with hamF12 medium containing 0.125 mg/mL G418 at 5 hr after the transfection medium, continuous overnight culture at 25°C, and culturing for 2 days at 37°C. Thereafter, the cells were used for the following calculation of the mutation introduction efficiency.

### <Calculation of mutation introduction efficiency>

The outline of the calculation of the mutation introduction efficiency is shown in Fig. 2. HPRT (Hypoxanthine-guanine phosophoribosyltransferase) is one of the purine metabolic enzymes, and the cells with destroyed HPRT gene acquires resistance to 6-TG (6-thioguanine). To calculate mutation introduction efficiency of HPRT gene, the cells were detached from plastic by using trypsin-EDTA (Life Technologies, Carlsbad, CA, USA) and 100-500 cells were spread on a dish containing hamF12 medium containing G418 or G418 + 5 g/mL 6-TG (Tokyo Chemical Industry, Tokyo, Japan). Seven days later, the number of resistant colonies was counted. The mutation introduction efficiency was calculated as a ratio of 6TG resistant colonies to the G418 resistant colonies.

### <Sequence analysis>

For sequence analysis, G418 and 6TG resistant colonies were treated with trypsin and pelletized by centrifugation. According to manufacturer's instructions, genomic DNA was extracted from the pellets by using NucleoSpin Tissue XS kit (Macherey-Nagel, Duren, Germany). PCR fragments containing the targeted site of HPRT was amplified from genomic DNA by using the forward primer (ggctacatagagggatcctgtgtca; SEQ ID NO: 18) and the reverse primer (acagtagctcttcagtctgataaaa; SEQ ID NO: 19). The PCR product was TA cloned into Escherichia coli (E. coli) vector and analyzed by the Sanger method.

### <Nucleic acid operation>

DNA was processed or constructed by any of PCR method, restriction enzyme treatment, ligation, Gibson Assembly method, and artificial chemical synthesis. The plasmid was amplified with Escherichia coli strain XL-10 gold or DH5α and introduced into the cells by the lipofection method.

### <Construct>

The outline of the genome editing plasmid vector used in the Example is shown in Fig. 1. Using pcDNA3.1 vector as a base, a vector used for gene transfer by transfection into CHO cells was constructed. A nuclear localization signal (ccc aag aag aag agg aag gtg; SEQ ID NO: 11 (PKKKRKV; encoding SEQ ID NO: 12)) which was added to Streptococcus pyogenes-derived Cas9 gene ORF having a codon optimized for eucaryon expression (SEQ ID NO: 3 (encoding SEQ ID NO: 4)), the resulting construct was ligated to the downstream of a CMV promoter via a linker sequence, a deaminase gene (Petromyzon marinus Petromyzon marinus-derived PmCDA1) ORF having a codon optimized for human cell expression (SEQ ID NO: 1 (encoding SEQ ID NO: 2)) was added thereto, and then the obtained product was expressed as a fusion protein. In addition, a construct for fusion expression of Ugi gene (PBS2-derived Ugi was codon-optimized for eukaryotic cell expression: SEQ ID NO: 5 (encoding SEQ ID NO: 6)) was also produced. A drug resistant gene (NeoR: G418 resistant gene) was also ligated via a sequence encoding 2A peptide (gaa ggc agg gga agc ctt ctg act tgt ggg gat gtg gaa gaa aac cct ggt cca; SEQ ID NO: 13 (encoding EGRGSLLTCGDVEENPGP; SEQ ID NO: 14)). As the linker sequence, 2xGS linker (two repeats of ggt gga gga ggt tct; SEQ ID NO: 15 (encoding GGGGS; SEQ ID NO: 16)) was used. As the terminator, SV40 poly A signal terminator (SEQ ID NO: 17) was ligated.

In Cas9, mutant Cas9 (nCas9) into which a mutation to convert the 10th aspartic acid to alanine (D10A, corresponding DNA sequence mutation a29c) was introduced and mutant Cas9 (dCas9) into which a mutation to convert the 840th histidine to alanine (H840A, corresponding DNA sequence mutation ca2518 gc) was further introduced were used to remove cleavage ability of each or both sides of DNA strand.

gRNA was placed between the H1 promoter (SEQ ID NO: 10) and the poly T signal (tttttt) as a chimeric structure with tracrRNA (derived from Streptococcus pyogenes; SEQ ID NO: 9) and incorporated into a plasmid vector for expressing the above-mentioned deaminase gene and the like. As the gRNA-targeting base sequence, the 16th - 34th sequence (ccgagatgtcatgaaagaga; SEQ ID NO: 7) (site 1) from the start point of exon3 of the HPRT gene, and a complementary strand sequence (ccatgacggaatcggtcggc; SEQ ID NO: 8) (site 2R) to the -15th - 3rd sequence from the start point of exon1 of the HPRT gene were used. They were introduced into the cell, expressed in the cells to form a complex of gRNA-tracrRNA and Cas9-PmCDA1 or Cas9-PmCDA1-Ugi.

### Example 1 Various genome editing plasmids and evaluation of mutation introduction efficiency by conditions

The evaluation results of various genome editing plasmids and mutation introduction efficiency by conditions are shown in Table 1. In Example 1, site 1 (SEQ ID NO: 7) was used as the gRNA-targeting base sequence for all those not described as site 2R.

**[Table 1]**

| | Modifier plasmid | Transformants | mutation frequency |
|---|---|---|---|
| | | 6-TG resisntant | |
| | Cas | 341 | 96.2% |
| | | 328 | |
| | nCas (D10A)-2A-Neo | 4745 | 0.06% |
| | | 3 | |
| | nCas-PmCDA1-2A-Neo | 282 | 35.9% |
| | | 101 | |
| | dCas-PmCDA1-2A-Neo | 384 | 2.08% |
| | | 8 | |
| | dCas-2A-Neo site 1 | 8066 | 0% |
| | | 0 | |
| | dCas-2A-Neo site2R | 6241 | 0% |
| | | 0 | |
| | Neo only | 15180 | 0% |
| | | 0 | |
| +25°C pulse | dCas-2A-Neo | 8900 | 0% |
| | | 0 | |
| | nCas-PmCDA1-2A-Neo | 480 | 61.9% |
| | | 297 | |
| | dCas-PmCDA1-2A-Neo | 240 | 12.5% |
| | | 30 | |
| +Ugi | nCas-PmCDA1-2A-Neo | 723 | 91.0% |
| | | 658 | |
| | dCas-PmCDA1-2A-Neo | 823 | 86.2% |
| | | 709 | |

A plasmid using nCas9 as mutant Cas9 (nCas-PmCDA1-2A-Neo) showed mutation introduction efficiency of 35.9%, and a plasmid using dCas9 as mutant Cas9 mutant (dCas-PmCDA1-2A-Neo) showed mutation introduction efficiency of 2.08%. On the other hand, in cases using a plasmid with Ugi ligated to PmCDA1, a plasmid using nCas9 as mutant Cas9 (+Ugi nCas-PmCDA1-2A-Neo) showed mutation introduction efficiency of 91.0%, and a plasmid using dCas9 as mutant Cas9 (+Ugi dCas-PmCDA1-2A-Neo) showed mutation introduction efficiency of 86.2%. Therefore, it was shown that mutation introduction efficiency is significantly improved by fusion expression of Ugi protein which inhibits repair of deaminated bases, and particularly, one using dCas9 showed a striking increase in the mutation introduction efficiency improving effect by the combined use of Ugi. In Table 1, Cas shows a plasmid using Cas9 without introduction of mutation, nCas(D10A)-2A-Neo, dCas-2A-Neo site 1 and dCas-2A-Neo site 2R show plasmids without ligation of a nucleic acid base converting enzyme, and they were each used as a control.

In addition, it was shown that the cells cultured temporarily (overnight) at a low temperature of 25°C (+25°C pulse) after transfection and using any of nCas9 (nCas-PmCDA1-2A-Neo) and dCas9 (dCas-PmCDA1-2A-Neo) exhibit significantly improved mutation introduction efficiency (61.9% and 12.5%, respectively). In Table 1, dCas-2A-Neo shows a plasmid without fusion with nucleic acid base converting enzyme and used as a control.

From the above, it was shown that, according to the genome editing technique of the present invention, the mutation introduction efficiency is strikingly improved as compared to the conventional genome editing techniques using nucleic acid base converting enzymes.

### Example 2 Analysis of mutation introduction pattern

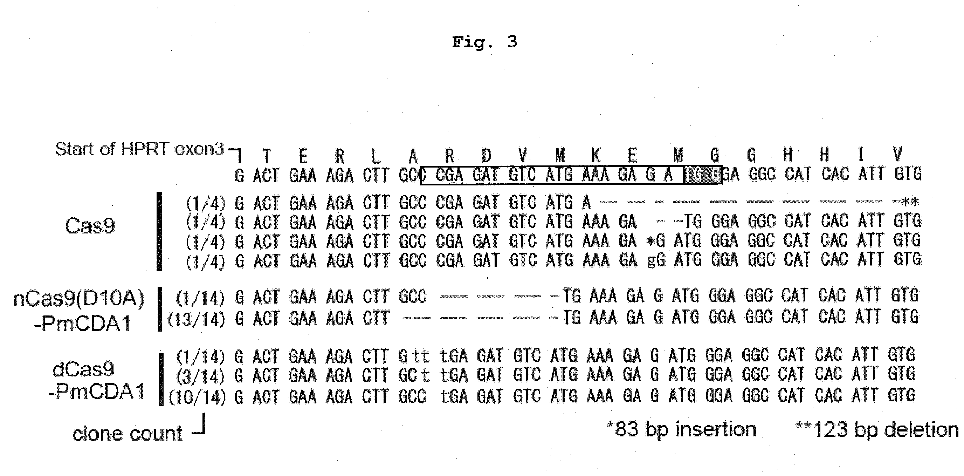

Genome DNA was extracted from the obtained mutation introduction colonies, the target region of the HPRT gene was amplified by PCR, TA cloning was performed, and sequence analysis was performed. The results are shown in Fig. 3. The editing vectors used were Cas9, nCas9(D10A)-PmCDA1 and dCas9-PmCDA1, the base excision repair inhibitor was not expressed, and the colonies from the cells cultured at 37°C were used. In the Figure, TGG enclosed in a black box shows PAM sequence.

In Cas9 free of introduction of mutation, large deletions and insertions centered on directly above the PAM sequence were observed. On the other hand, in nCas9 (D10A)-PmCDA1, a small scale deletion of about dozen bases was observed, and the region thereof contained a deamination target base. In dCas9-PmCDA1, a mutation from C to T was observed at 19 to 21 bases upstream of the PAM sequence, and a single base pinpoint mutation was introduced in 10 clones in total 14 clones subjected to sequence analysis.

From the above, it was shown that pinpoint mutation introduction is possible even when a genome editing technique using a nucleic acid base converting enzyme is applied to mammalian cells.

### Example 3 Study using other mammalian cell

Using HEK293T cell, which is derived from human fetal kidney, mutation introduction efficiency was evaluated. The same vector as in Example 1 was used as a vector except that the gRNA target base sequence was the sequence of the EMX1 gene (shown in SEQ ID NO: 21) described in "Tsai S.Q. et al., (2015) Nat Biotechnol., 33(2): 187-197" and the off-target candidate sequences 1 to 4 (respectively correspond to the sequences of Emx 1 off target 1 - Emx 1 off target 4 in Tables 2, 3 and shown in SEQ ID NOs: 22 - 25). The vector was introduced into HEK293T cells by transfection. Without selection of the cells, the whole cells were recovered two days later and genomic DNA was extracted. Culture conditions other than cell selection and period up to total cell recovery and transfection conditions were the same as those of the above-mentioned CHO-K1 cells. Then, according to the method described in "Nishida K. et al. (2016) Science, 6: 353(6305)", the regions containing respective targets were amplified by PCR using the primers shown in Table 2, and mutation introduction pattern was analyzed using a next-generation sequencer. The results are shown in Table 3. In the Table, the number under the sequence shows a substitution rate (%) of the nucleotide.

**[Table 2]**

| target name | SEQ ID NO: | Primer name | Primer sequence (5'→3') |
|---|---|---|---|
| Emx1 | 26 | TA501 EMX 1st-3 | GTAGTCTGGCTGTCACAGGCCATACTCTTCCACAT |
| | 27 | TA575 EMX 1st-6 | GTGGGTGACCCACCCAAGCAGCAGGCTCTCCACCA |
| | 28 | TA576 EMX 2nd-3 | TCTTTCCCTACACGACGCTCTTCCGATCTACTTAGCTGGAGTGTGGAGGCTATCTTGGC |
| | 29 | TA410 EMX 2nd-2 | GTGACTGGAGTTCAGACGTGTGCTCTTCCGATCTGGCTAGGGACTGGCCAGAGTCCAGC |
| Emx1 off target 1 | 30 | TA502 EMX-off1 1st-3 | CTGCCCATATCCACCACAAGCAAGTTAGTCATCAA |
| | 31 | TA412 EMX-off1 1st-2 | AATCAAAATCTCTATGTGTGGGGCACAGGG |
| | 32 | TA413 EMX-off1 2nd-1 | TCTTTCCCTACACGACGCTCTTCCGATCTCATTGGCTAGAATTCAGACTTCAAG |
| | 33 | TA414 EMX-off1 2nd-2 | GTGACTGGAGTTCAGACGTGTGCTCTTCCGATCTATGAGGGAGATGTACTCTCAAGTGA |
| Emx1 off target 2 | 34 | TA503 EMX-off2 1st-3 | CATGTTCCCTCACCCTTGGCATCTACACACTTTCT |
| | 35 | TA416 EMX-off2 1st-2 | TAGTTTACCCTGAGGCAATATCTGACTCCA |
| | 36 | TA417 EMX-off2 2nd-1 | TCTTTCCCTACACGACGCTCTTCCGATCTTCATTTTCAAATGCCTATTGAGCGG |
| | 37 | TA418 EMX-off2 2nd-2 | GTGACTGGAGTTCAGACGTGTGCTCTTCCGATCTAAGGCTCCTTGCCTTTACATATAGG |
| Emx1 off target 3 | 38 | TA419 EMX-off3 1st-1 | TCACTTTTGTCAATTCATGCCACCATCAGT |
| | 39 | TA420 EMX-off3 1st-2 | GCCACCTCCACTCTGCCAGGAATAGGTTCA |
| | 40 | TA421 EMX-off3 2nd-1 | TCTTTCCCTACACGACGCTCTTCCGATCTATGGACTGTCCTGTGAGCCCGTGGC |
| | 41 | TA422 EMX-off3 2nd-2 | GTGACTGGAGTTCAGACGTGTGCTCTTCCGATCTCTCGGTGGCCTGCAAGTGGAAAGCC |
| Emx1 off target 4 | 42 | TA423 EMX-off4 1st-1 | GGGACCACTTGAAGTGAGTAAAATTATAGG |
| | 43 | TA424 EMX-off4 1st-2 | CCCAGCTGTTGCTAGCTTATGGCCAGTCCT |
| | 44 | TA425 EMX-off4 2nd-1 | TCTTTCCCTACACGACGCTCTTCCGATCTCACTGCCTTTCGGGCTAGCCTCCAA |
| | 45 | TA426 EMX-off4 2nd-2 | GTGACTGGAGTTCAGACGTGTGCTCTTCCGATCTTAGATGTTAATAGGTTATTGGGGTG |

| | | | |
|---|---|---|---|
| Fragments amplified from genomic DNA with a pair of 1st primers were amplified again with a pair of 2nd primers to add an adapter sequence for NGS. | | | |

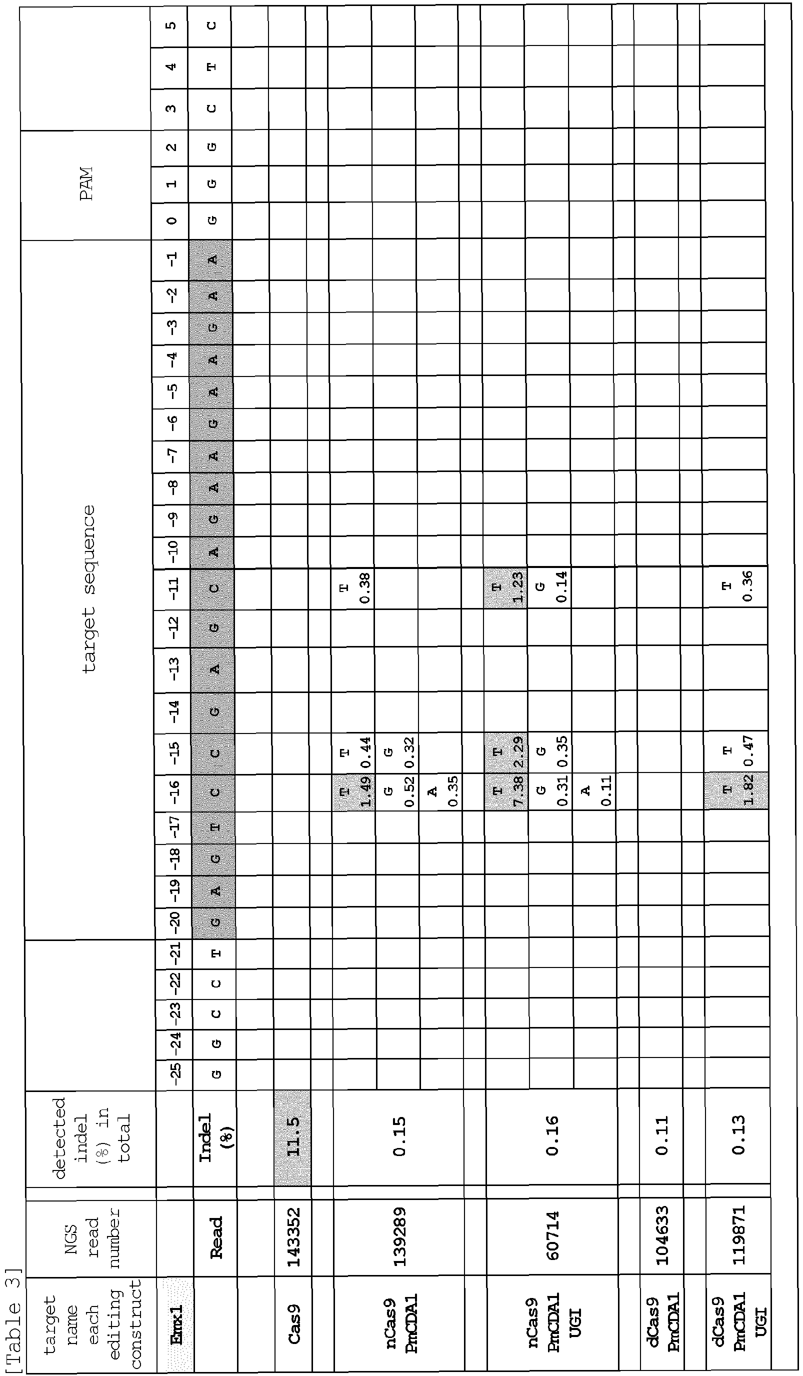

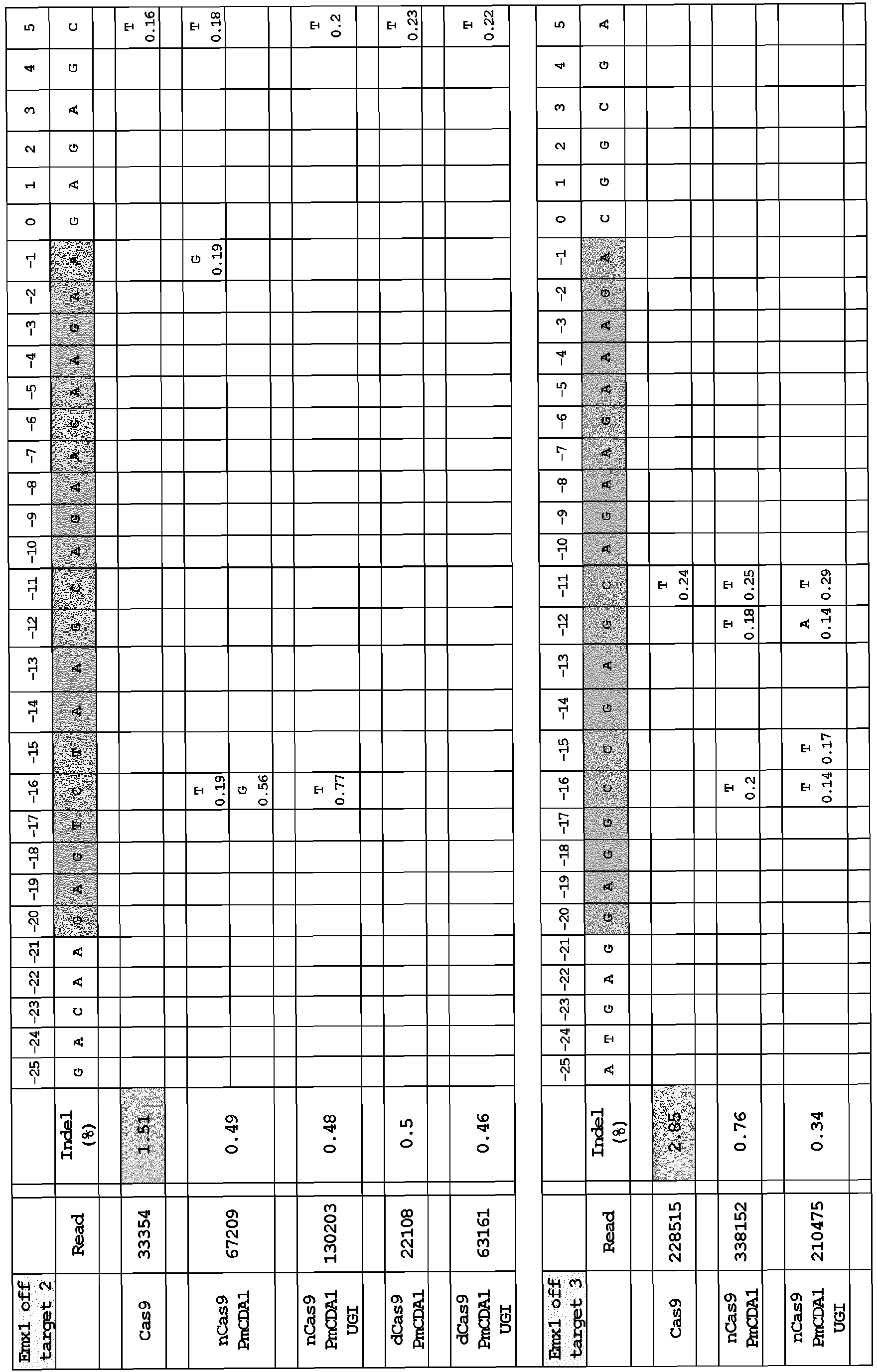

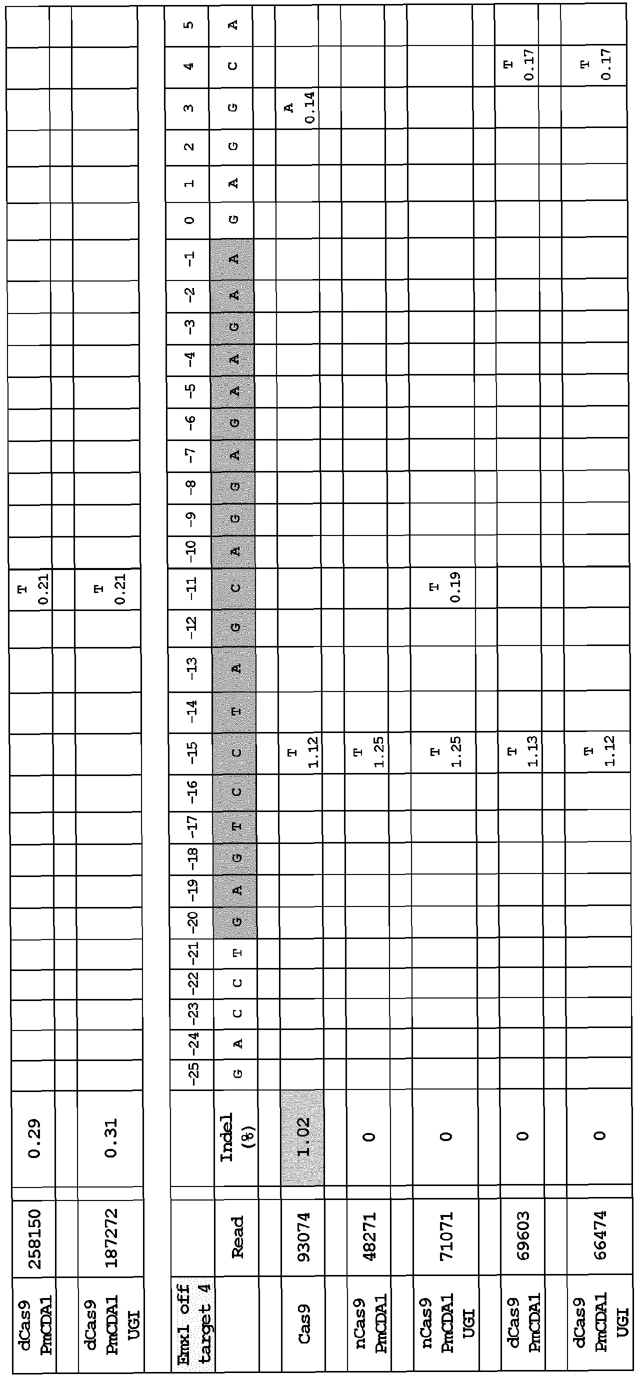

From Table 3, a mutation introduction efficiency improving effect by the combined use of UGI was found even when human cells were used. Furthermore, it was suggested that the ratio of off-target mutation rate to on-target can be lowered, namely, the risk of off-target mutation can be suppressed. To be specific, when nCas9-PmCDA1-UGI was used, for example, the ratio of the substitution rate of cytosine at the corresponding position of the off-target candidate to the substitution rate of the -16th cytosine in the target sequence of EMX1 was not more than 1/10. When nCas9-PmCDA1-UGI was used, the mutation rate was improved in the target sequence of EMX1 as compared to that of nCas9-PmCDA1 without combined use of UGI; however, in the off-target candidate sequence, the mutation rate showed almost no difference and off-target mutation was suppressed. Similarly, when dCas9-PmCDA1-UGI was used, the mutation rate was improved in the target sequence of EMX1 as compared to that of dCas9-PmCDA1 without combined use of UGI; however, the mutation rate showed almost no difference in the off-target candidate sequence and off-target mutation was suppressed. As for the -15th cytosine in the off-target candidate sequence 4 (sequence name: Emx1 off target 4) in Table 3, substitution occurred at the same rate irrespective of the vector used. Since substitution is found similarly in Cas9 as well, these substitutions were highly possibly caused by sequencing errors.

### [Industrial Applicability]

The present invention makes it possible to safely introduce site specific mutation into any species highly efficiently without accompanying insertion of a foreign DNA or double-stranded DNA breaks, and is extremely useful.

## Claims

1. A method of modifying a targeted site of a double-stranded DNA, comprising a step of introducing a complex wherein a nucleic acid sequence-recognizing module that specifically binds to a target nucleotide sequence in a given double-stranded DNA and PmCDA1 are bonded, into a mammalian cell containing the double-stranded DNA, and culturing the cell at a temperature of 20°C to 35°C at least temporarily to convert one or more nucleotides in the targeted site to other one or more nucleotides or delete one or more nucleotides, or insert one or more nucleotides into said targeted site, without cleaving at least one strand of said double-stranded DNA in the targeted site,
wherein the nucleic acid sequence-recognizing module is a CRISPR-Cas system wherein at least one DNA cleavage ability of Cas is inactivated.

2. The method according to claim 1, wherein said Cas is deficient in two DNA cleavage abilities.

3. The method according to claim 1 or 2, wherein the temperature is 20°C to 25°C.

4. The method according to claim 1 or 2, wherein the temperature is 25°C.

5. The method according to any one of claims 1 to 4, wherein the double-stranded DNA is contacted with the complex by introducing a nucleic acid encoding the complex into a mammalian cell having the double-stranded DNA.

6. A method of modifying a targeted site of a double-stranded DNA, comprising a step of contacting a complex wherein a nucleic acid sequence-recognizing module that specifically binds to a target nucleotide sequence in a given double-stranded DNA, a nucleic acid base converting enzyme and a base excision repair inhibitor are bonded, with said double-stranded DNA to convert one or more nucleotides in the targeted site to other one or more nucleotides or delete one or more nucleotides, or insert one or more nucleotides into said targeted site, without cleaving at least one strand of said double-stranded DNA in the targeted site,
wherein the nucleic acid sequence-recognizing module is a CRISPR-Cas system wherein at least one DNA cleavage ability of Cas is inactivated,
wherein the nucleic acid base converting enzyme is PmCDA1 encoded by a nucleotide sequence having codon optimized for a host cell.

7. The method according to claim 6, wherein said Cas is deficient in two DNA cleavage abilities.

8. The method according to claim 6 or 7, wherein the base excision repair inhibitor is a uracil DNA glycosylase inhibitor.

9. The method according to claim 6, wherein the host cell is a mammalian cell.

10. The method according to any one of claims 6 to 9, wherein the double-stranded DNA is contacted with the complex by introducing a nucleic acid encoding the complex into a cell having the double-stranded DNA.

11. A nucleic acid-modifying enzyme complex wherein a nucleic acid sequence-recognizing module that specifically binds to a target nucleotide sequence in a given double-stranded DNA, a nucleic acid base converting enzyme and a base excision repair inhibitor are bonded, which complex converts one or more nucleotides in the targeted site to other one or more nucleotides or deletes one or more nucleotides, or inserts one or more nucleotides into said targeted site, without cleaving at least one strand of said double-stranded DNA in the targeted site,
wherein the nucleic acid sequence-recognizing module is a CRISPR-Cas system wherein at least one DNA cleavage ability of Cas is inactivated, and
wherein the nucleic acid base converting enzyme is PmCDA1 encoded by a nucleotide sequence having codon optimized for a host cell.

12. The nucleic acid-modifying enzyme complex according to claim 11, wherein the host cell is a mammalian cell.

13. A nucleic acid encoding the nucleic acid-modifying enzyme complex according to claim 11 or 12.

## Patentansprüche

1. Verfahren zur Modifizierung einer Zielstelle einer doppelsträngigen DNA, das Folgendes umfasst: einen Schritt der Einführung eines Komplexes, wobei ein Nukleinsäuresequenzerfassungsmodul, das spezifisch an eine Zielnukleotidsequenz in einer gegebenen doppelsträngigen DNA bindet, und PmCDA1 in eine Säugetierzelle gebunden sind, die die doppelsträngige DNA enthält, und Kultivieren der Zelle bei einer Temperatur von 20 °C bis 35 °C zumindest zeitweise, um ein oder mehrere Nukleotide an der Zielstelle in ein oder mehrere andere Nukleotide umzuwandeln oder ein oder mehrere Nukleotide zu entfernen, oder ein oder mehrere Nukleotide in die Zielstelle einzufügen, ohne mindestens einen Strang der doppelsträngigen DNA an der Zielstelle zu spalten,
wobei das Nukleinsäuresequenzerfassungsmodul ein CRISPR-Cas-System ist, wobei mindestens eine DNA-Spaltungsfähigkeit von Cas inaktiviert ist.

2. Verfahren nach Anspruch 1, wobei das Cas in zwei DNA-Spaltungsfähigkeiten defizitär ist.

3. Verfahren nach Anspruch 1 oder 2, wobei die Temperatur 20 °C bis 25 °C beträgt.

4. Verfahren nach Anspruch 1 oder 2, wobei die Temperatur 25 °C beträgt.

5. Verfahren nach einem der Ansprüche 1 bis 4, wobei die doppelsträngige DNA mit dem Komplex in Kontakt gebracht wird, indem eine Nukleinsäure, die für den Komplex codiert, in eine Säugetierzelle mit der doppelsträngigen DNA eingeführt wird.

6. Verfahren zur Modifizierung einer Zielstelle einer doppelsträngigen DNA, das einen Schritt des Inkontaktbringens eines Komplexes umfasst, wobei ein Nukleinsäuresequenzerfassungsmodul, das spezifisch an eine Zielnukleotidsequenz in einer gegebenen doppelsträngigen DNA bindet, ein Nukleinsäurebasenumwandlungsenzym und ein Basenausscheidungsreparaturinhibitor mit der doppelsträngigen DNA gebunden sind, um ein oder mehrere Nukleotide an der Zielstelle in ein oder mehrere andere Nukleotide umzuwandeln oder ein oder mehrere Nukleotide zu entfernen, oder ein oder mehrere Nukleotide in die Zielstelle einzufügen, ohne mindestens einen Strang der doppelsträngigen DNA an der Zielstelle zu spalten,
wobei das Nukleinsäuresequenzerfassungsmodul ein CRISPR-Cas-System ist, wobei mindestens eine DNA-Spaltungsfähigkeit von Cas inaktiviert ist.
wobei das Nukleinsäurebasenumwandlungsenzym PmCDAl ist, das durch eine Nukleotidsequenz mit einem für eine Wirtszelle optimierten Codon codiert wird.

7. Verfahren nach Anspruch 6, wobei das Cas in zwei DNA-Spaltungsfähigkeiten defizitär ist.

8. Verfahren nach Anspruch 6 oder 7, wobei der Basenausscheidungsreparaturinhibitor ein Uracil-DNA-Glycosylase-Inhibitor ist.

9. Verfahren nach Anspruch 6, wobei die Wirtszelle eine Säugetierzelle ist.

10. Verfahren nach einem der Ansprüche 6 bis 9, wobei die doppelsträngige DNA mit dem Komplex in Kontakt gebracht wird, indem eine Nukleinsäure, die für den Komplex codiert, in eine Zelle mit der doppelsträngigen DNA eingeführt wird.

11. Nukleinsäuremodifizierender Enzymkomplex, wobei ein Nukleinsäuresequenzerfassungsmodul, das spezifisch an eine Zielnukleotidsequenz in einer gegebenen doppelsträngigen DNA bindet, ein Nukleinsäurebasenumwandlungsenzym und ein Basenausscheidungsreparaturinhibitor gebunden sind, wobei der Komplex ein oder mehrere Nukleotide an der Zielstelle in ein oder mehrere andere Nukleotide umwandelt oder ein oder mehrere Nukleotide entfernt, oder ein oder mehrere Nukleotide in die Zielstelle einfügt, ohne mindestens einen Strang der doppelsträngigen DNA an der Zielstelle zu spalten,
wobei das Nukleinsäuresequenzerfassungsmodul ein CRISPR-Cas-System ist, wobei mindestens eine DNA-Spaltungsfähigkeit von Cas inaktiviert ist, und
wobei das Nukleinsäurebasenumwandlungsenzym PmCDAl ist, das durch eine Nukleotidsequenz mit einem für eine Wirtszelle optimierten Codon codiert wird.

12. Nukleinsäuremodifizierender Enzymkomplex nach Anspruch 11, wobei die Wirtszelle eine Säugetierzelle ist.

13. Nukleinsäure, die für den nukleinsäuremodifizierenden Enzymkomplex nach Anspruch 11 oder 12 codiert.

## Revendications

1. Procédé de modification d'un site ciblé d'un ADN double brin, comprenant une étape d'introduction d'un complexe dans lequel un module de reconnaissance de séquence d'acide nucléique qui se lie spécifiquement à une séquence nucléotidique cible dans un ADN double brin donné et PmCDA1 sont liés, dans une cellule de mammifère contenant l'ADN double brin, et de culture de la cellule à une température de 20°C à 35°C au moins temporairement pour convertir un ou plusieurs nucléotides dans le site ciblé en un ou plusieurs autres nucléotides ou supprimer un ou plusieurs nucléotides, ou insérer un ou plusieurs nucléotides dans ledit site ciblé, sans cliver au moins un brin dudit ADN double brin dans le site ciblé,
dans lequel le module de reconnaissance de séquence d'acide nucléique est un système CRISPR-Cas dans lequel au moins une capacité de clivage d'ADN de Cas est inactivée.

2. Procédé selon la revendication 1, dans lequel ledit Cas est déficient en deux capacités de clivage d'ADN.

3. Procédé selon la revendication 1 ou 2, dans lequel la température est de 20°C à 25°C.

4. Procédé selon la revendication 1 ou 2, dans lequel la température est de 25°C.

5. Procédé selon l'une quelconque des revendications 1 à 4, dans lequel l'ADN double brin est mis en contact avec le complexe en introduisant un acide nucléique codant pour le complexe dans une cellule de mammifère ayant l'ADN double brin.

6. Procédé de modification d'un site ciblé d'un ADN double brin, comprenant une étape de mise en contact d'un complexe dans lequel un module de reconnaissance de séquence d'acide nucléique qui se lie spécifiquement à une séquence nucléotidique cible dans un ADN double brin donné, une enzyme de conversion de base d'acide nucléique et un inhibiteur de réparation par excision de base sont liés, audit ADN double brin pour convertir un ou plusieurs nucléotides dans le site ciblé en un ou plusieurs autres nucléotides ou supprimer un ou plusieurs nucléotides, ou insérer un ou plusieurs nucléotides dans ledit site ciblé site, sans cliver au moins un brin dudit ADN double brin dans le site ciblé,
dans lequel le module de reconnaissance de séquence d'acide nucléique est un système CRISPR-Cas dans lequel au moins une capacité de clivage d'ADN de Cas est inactivée,
dans lequel l'enzyme de conversion de base d'acide nucléique est PmCDA1 codée par une séquence nucléotidique ayant un codon optimisé pour une cellule hôte.

7. Procédé selon la revendication 6, dans lequel ledit Cas est déficient en deux capacités de clivage d'ADN.

8. Procédé selon la revendication 6 ou 7, dans lequel l'inhibiteur de réparation par excision de base est un inhibiteur d'uracile ADN glycosylase.

9. Procédé selon la revendication 6, dans lequel la cellule hôte est une cellule de mammifère.

10. Procédé selon l'une quelconque des revendications 6 à 9, dans lequel l'ADN double brin est mis en contact avec le complexe en introduisant un acide nucléique codant pour le complexe dans une cellule ayant l'ADN double brin.

11. Complexe enzymatique de modification d'acide nucléique dans lequel un module de reconnaissance de séquence d'acide nucléique qui se lie spécifiquement à une séquence nucléotidique cible dans un ADN double brin donné, une enzyme de conversion de base d'acide nucléique et un inhibiteur de réparation par excision de base sont liés, lequel complexe convertit un ou plusieurs nucléotides dans le site ciblé en un ou plusieurs autres nucléotides ou supprime un ou plusieurs nucléotides, ou insère un ou plusieurs nucléotides dans ledit site ciblé, sans cliver au moins un brin dudit ADN double brin dans le site ciblé,
dans lequel le module de reconnaissance de séquence d'acide nucléique est un système CRISPR-Cas dans lequel au moins une capacité de clivage d'ADN de Cas est inactivée, et dans lequel l'enzyme de conversion de base d'acide nucléique est PmCDA1 codée par une séquence nucléotidique ayant un codon optimisé pour une cellule hôte.

12. Complexe enzymatique de modification d'acide nucléique selon la revendication 11, dans lequel la cellule hôte est une cellule de mammifère.

13. Acide nucléique codant pour le complexe enzymatique de modification d'acide nucléique selon la revendication 11 ou 12.


### SEQUENCE LISTING

<110> NATIONAL UNIVERSITY CORPORATION KOBE UNIVERSITY
<120> Method for improving mutation introduction efficiency in genomic sequence modification technology, and molecular complex for use in same
<130> 092589
<150> JP 2016-085631
   <151> 2016-04-21
<160> 60
<170> PatentIn version 3.5
<210> 1
   <211> 624
   <212> DNA
   <213> Artificial Sequence
<220>
   <223> PmCDA1 CDS optimized for human cell expression
<220>
   <221> CDS
   <222> (1)..(624)
<400> 1
   atg aca gac gcc gag tac gtg cgc att cat gag aaa ctg gat att tac        48
   Met Thr Asp Ala Glu Tyr Val Arg Ile His Glu Lys Leu Asp Ile Tyr
   1               5                   10                  15
   acc ttc aag aag cag ttc ttc aac aac aag aaa tct gtg tca cac cgc        96
   Thr Phe Lys Lys Gln Phe Phe Asn Asn Lys Lys Ser Val Ser His Arg
               20                  25                  30
   tgc tac gtg ctg ttt gag ttg aag cga agg ggc gaa aga agg gct tgc       144
   Cys Tyr Val Leu Phe Glu Leu Lys Arg Arg Gly Glu Arg Arg Ala Cys
           35                  40                  45
   ttt tgg ggc tat gcc gtc aac aag ccc caa agt ggc acc gag aga gga       192
   Phe Trp Gly Tyr Ala Val Asn Lys Pro Gln Ser Gly Thr Glu Arg Gly
       50                  55                  60
   ata cac gct gag ata ttc agt atc cga aag gtg gaa gag tat ctt cgg       240
   Ile His Ala Glu Ile Phe Ser Ile Arg Lys Val Glu Glu Tyr Leu Arg
   65                  70                  75                  80
   gat aat cct ggg cag ttt acg atc aac tgg tat tcc agc tgg agt cct       288
   Asp Asn Pro Gly Gln Phe Thr Ile Asn Trp Tyr Ser Ser Trp Ser Pro
                   85                  90                  95
   tgc gct gat tgt gcc gag aaa att ctg gaa tgg tat aat cag gaa ctt       336
   Cys Ala Asp Cys Ala Glu Lys Ile Leu Glu Trp Tyr Asn Gln Glu Leu
               100                 105                 110
   cgg gga aac ggg cac aca ttg aaa atc tgg gcc tgc aag ctg tac tac       384
   Arg Gly Asn Gly His Thr Leu Lys Ile Trp Ala Cys Lys Leu Tyr Tyr
           115                 120                 125
   gag aag aat gcc cgg aac cag ata gga ctc tgg aat ctg agg gac aat       432
   Glu Lys Asn Ala Arg Asn Gln Ile Gly Leu Trp Asn Leu Arg Asp Asn
       130                 135                 140
   ggt gta ggc ctg aac gtg atg gtt tcc gag cac tat cag tgt tgt cgg       480
   Gly Val Gly Leu Asn Val Met Val Ser Glu His Tyr Gln Cys Cys Arg
   145                 150                 155                 160
   aag att ttc atc caa agc tct cat aac cag ctc aat gaa aac cgc tgg       528
   Lys Ile Phe Ile Gln Ser Ser His Asn Gln Leu Asn Glu Asn Arg Trp
                   165                 170                 175
   ttg gag aaa aca ctg aaa cgt gcg gag aag cgg aga tcc gag ctg agc       576
   Leu Glu Lys Thr Leu Lys Arg Ala Glu Lys Arg Arg Ser Glu Leu Ser
               180                 185                 190
   atc atg atc cag gtc aag att ctg cat acc act aag tct cca gcc gtt       624
   Ile Met Ile Gln Val Lys Ile Leu His Thr Thr Lys Ser Pro Ala Val
           195                 200                 205
<210> 2
   <211> 208
   <212> PRT
   <213> Artificial Sequence
<220>
   <223> Synthetic Construct
<400> 2
   Met Thr Asp Ala Glu Tyr Val Arg Ile His Glu Lys Leu Asp Ile Tyr
   1 5 10 15
   Thr Phe Lys Lys Gln Phe Phe Asn Asn Lys Lys Ser Val Ser His Arg
   20 25 30
   Cys Tyr Val Leu Phe Glu Leu Lys Arg Arg Gly Glu Arg Arg Ala Cys
   35 40 45
   Phe Trp Gly Tyr Ala Val Asn Lys Pro Gln Ser Gly Thr Glu Arg Gly
   50 55 60
   Ile His Ala Glu Ile Phe Ser Ile Arg Lys Val Glu Glu Tyr Leu Arg
   65 70 75 80
   Asp Asn Pro Gly Gln Phe Thr Ile Asn Trp Tyr Ser Ser Trp Ser Pro
   85 90 95
   Cys Ala Asp Cys Ala Glu Lys Ile Leu Glu Trp Tyr Asn Gln Glu Leu
   100 105 110
   Arg Gly Asn Gly His Thr Leu Lys Ile Trp Ala Cys Lys Leu Tyr Tyr
   115 120 125
   Glu Lys Asn Ala Arg Asn Gln Ile Gly Leu Trp Asn Leu Arg Asp Asn
   130 135 140
   Gly Val Gly Leu Asn Val Met Val Ser Glu His Tyr Gln Cys Cys Arg
   145 150 155 160
   Lys Ile Phe Ile Gln Ser Ser His Asn Gln Leu Asn Glu Asn Arg Trp
   165 170 175
   Leu Glu Lys Thr Leu Lys Arg Ala Glu Lys Arg Arg Ser Glu Leu Ser
   180 185 190
   Ile Met Ile Gln Val Lys Ile Leu His Thr Thr Lys Ser Pro Ala Val
   195 200 205
<210> 3
   <211> 4116
   <212> DNA
   <213> Artificial Sequence
<220>
   <223> Streptococcus pyogenes-derived Cas9 CDS optimized for eucaryotic cell expression
<220>
   <221> CDS
   <222> (1)..(4116)
<400> 3
   atg gac aag aag tac tcc att ggg ctc gat atc ggc aca aac agc gtc        48
   Met Asp Lys Lys Tyr Ser Ile Gly Leu Asp Ile Gly Thr Asn Ser Val
   1               5                   10                  15
   ggt tgg gcc gtc att acg gac gag tac aag gtg ccg agc aaa aaa ttc        96
   Gly Trp Ala Val Ile Thr Asp Glu Tyr Lys Val Pro Ser Lys Lys Phe
               20                  25                  30
   aaa gtt ctg ggc aat acc gat cgc cac agc ata aag aag aac ctc att       144
   Lys Val Leu Gly Asn Thr Asp Arg His Ser Ile Lys Lys Asn Leu Ile
           35                  40                  45
   ggc gcc ctc ctg ttc gac tcc ggg gag acg gcc gaa gcc acg cgg ctc       192
   Gly Ala Leu Leu Phe Asp Ser Gly Glu Thr Ala Glu Ala Thr Arg Leu
       50                  55                  60
   aaa aga aca gca cgg cgc aga tat acc cgc aga aag aat cgg atc tgc       240
   Lys Arg Thr Ala Arg Arg Arg Tyr Thr Arg Arg Lys Asn Arg Ile Cys
   65                  70                  75                  80
   tac ctg cag gag atc ttt agt aat gag atg gct aag gtg gat gac tct       288
   Tyr Leu Gln Glu Ile Phe Ser Asn Glu Met Ala Lys Val Asp Asp Ser
                   85                  90                  95
   ttc ttc cat agg ctg gag gag tcc ttt ttg gtg gag gag gat aaa aag       336
   Phe Phe His Arg Leu Glu Glu Ser Phe Leu Val Glu Glu Asp Lys Lys
               100                 105                 110
   cac gag cgc cac cca atc ttt ggc aat atc gtg gac gag gtg gcg tac        384
   His Glu Arg His Pro Ile Phe Gly Asn Ile Val Asp Glu Val Ala Tyr
           115                 120                 125
   cat gaa aag tac cca acc ata tat cat ctg agg aag aag ctt gta gac        432
   His Glu Lys Tyr Pro Thr Ile Tyr His Leu Arg Lys Lys Leu Val Asp
       130                 135                 140
   agt act gat aag gct gac ttg cgg ttg atc tat ctc gcg ctg gcg cat        480
   Ser Thr Asp Lys Ala Asp Leu Arg Leu Ile Tyr Leu Ala Leu Ala His
   145                 150                 155                 160
   atg atc aaa ttt cgg gga cac ttc ctc atc gag ggg gac ctg aac cca        528
   Met Ile Lys Phe Arg Gly His Phe Leu Ile Glu Gly Asp Leu Asn Pro
                   165                 170                 175
   gac aac agc gat gtc gac aaa ctc ttt atc caa ctg gtt cag act tac        576
   Asp Asn Ser Asp Val Asp Lys Leu Phe Ile Gln Leu Val Gln Thr Tyr
               180                 185                 190
   aat cag ctt ttc gaa gag aac ccg atc aac gca tcc gga gtt gac gcc        624
   Asn Gln Leu Phe Glu Glu Asn Pro Ile Asn Ala Ser Gly Val Asp Ala
           195                 200                 205
   aaa gca atc ctg agc gct agg ctg tcc aaa tcc cgg cgg ctc gaa aac        672
   Lys Ala Ile Leu Ser Ala Arg Leu Ser Lys Ser Arg Arg Leu Glu Asn
       210                 215                 220
   ctc atc gca cag ctc cct ggg gag aag aag aac ggc ctg ttt ggt aat        720
   Leu Ile Ala Gln Leu Pro Gly Glu Lys Lys Asn Gly Leu Phe Gly Asn
   225                 230                 235                 240
   ctt atc gcc ctg tca ctc ggg ctg acc ccc aac ttt aaa tct aac ttc        768
   Leu Ile Ala Leu Ser Leu Gly Leu Thr Pro Asn Phe Lys Ser Asn Phe
                   245                 250                 255
   gac ctg gcc gaa gat gcc aag ctt caa ctg agc aaa gac acc tac gat        816
   Asp Leu Ala Glu Asp Ala Lys Leu Gln Leu Ser Lys Asp Thr Tyr Asp
               260                 265                 270
   gat gat ctc gac aat ctg ctg gcc cag atc ggc gac cag tac gca gac        864
   Asp Asp Leu Asp Asn Leu Leu Ala Gln Ile Gly Asp Gln Tyr Ala Asp
           275                 280                 285
   ctt ttt ttg gcg gca aag aac ctg tca gac gcc att ctg ctg agt gat        912
   Leu Phe Leu Ala Ala Lys Asn Leu Ser Asp Ala Ile Leu Leu Ser Asp
       290                 295                 300
   att ctg cga gtg aac acg gag atc acc aaa gct ccg ctg agc gct agt        960
   Ile Leu Arg Val Asn Thr Glu Ile Thr Lys Ala Pro Leu Ser Ala Ser
   305                 310                 315                 320
   atg atc aag cgc tat gat gag cac cac caa gac ttg act ttg ctg aag       1008
   Met Ile Lys Arg Tyr Asp Glu His His Gln Asp Leu Thr Leu Leu Lys
                   325                 330                 335
   gcc ctt gtc aga cag caa ctg cct gag aag tac aag gaa att ttc ttc       1056
   Ala Leu Val Arg Gln Gln Leu Pro Glu Lys Tyr Lys Glu Ile Phe Phe
               340                 345                 350
   gat cag tct aaa aat ggc tac gcc gga tac att gac ggc gga gca agc       1104
   Asp Gln Ser Lys Asn Gly Tyr Ala Gly Tyr Ile Asp Gly Gly Ala Ser
           355                 360                 365
   cag gag gaa ttt tac aaa ttt att aag ccc atc ttg gaa aaa atg gac      1152
   Gln Glu Glu Phe Tyr Lys Phe Ile Lys Pro Ile Leu Glu Lys Met Asp
       370                 375                 380
   ggc acc gag gag ctg ctg gta aag ctt aac aga gaa gat ctg ttg cgc      1200
   Gly Thr Glu Glu Leu Leu Val Lys Leu Asn Arg Glu Asp Leu Leu Arg
   385                 390                 395                 400
   aaa cag cgc act ttc gac aat gga agc atc ccc cac cag att cac ctg      1248
   Lys Gln Arg Thr Phe Asp Asn Gly Ser Ile Pro His Gln Ile His Leu
                   405                 410                 415
   ggc gaa ctg cac gct atc ctc agg cgg caa gag gat ttc tac ccc ttt      1296
   Gly Glu Leu His Ala Ile Leu Arg Arg Gln Glu Asp Phe Tyr Pro Phe
               420                 425                 430
   ttg aaa gat aac agg gaa aag att gag aaa atc ctc aca ttt cgg ata      1344
   Leu Lys Asp Asn Arg Glu Lys Ile Glu Lys Ile Leu Thr Phe Arg Ile
           435                 440                 445
   ccc tac tat gta ggc ccc ctc gcc cgg gga aat tcc aga ttc gcg tgg      1392
   Pro Tyr Tyr Val Gly Pro Leu Ala Arg Gly Asn Ser Arg Phe Ala Trp
       450                 455                 460
   atg act cgc aaa tca gaa gag acc atc act ccc tgg aac ttc gag gaa      1440
   Met Thr Arg Lys Ser Glu Glu Thr Ile Thr Pro Trp Asn Phe Glu Glu
   465                 470                 475                 480
   gtc gtg gat aag ggg gcc tct gcc cag tcc ttc atc gaa agg atg act      1488
   Val Val Asp Lys Gly Ala Ser Ala Gln Ser Phe Ile Glu Arg Met Thr
                   485                 490                 495
   aac ttt gat aaa aat ctg cct aac gaa aag gtg ctt cct aaa cac tct      1536
   Asn Phe Asp Lys Asn Leu Pro Asn Glu Lys Val Leu Pro Lys His Ser
               500                 505                 510
   ctg ctg tac gag tac ttc aca gtt tat aac gag ctc acc aag gtc aaa      1584
   Leu Leu Tyr Glu Tyr Phe Thr Val Tyr Asn Glu Leu Thr Lys Val Lys
           515                 520                 525
   tac gtc aca gaa ggg atg aga aag cca gca ttc ctg tct gga gag cag      1632
   Tyr Val Thr Glu Gly Met Arg Lys Pro Ala Phe Leu Ser Gly Glu Gln
       530                 535                 540
   aag aaa gct atc gtg gac ctc ctc ttc aag acg aac cgg aaa gtt acc      1680
   Lys Lys Ala Ile Val Asp Leu Leu Phe Lys Thr Asn Arg Lys Val Thr
   545                 550                 555                 560
   gtg aaa cag ctc aaa gaa gac tat ttc aaa aag att gaa tgt ttc gac      1728
   Val Lys Gln Leu Lys Glu Asp Tyr Phe Lys Lys Ile Glu Cys Phe Asp
                   565                 570                 575
   tct gtt gaa atc agc gga gtg gag gat cgc ttc aac gca tcc ctg gga      1776
   Ser Val Glu Ile Ser Gly Val Glu Asp Arg Phe Asn Ala Ser Leu Gly
               580                 585                 590
   acg tat cac gat ctc ctg aaa atc att aaa gac aag gac ttc ctg gac      1824
   Thr Tyr His Asp Leu Leu Lys Ile Ile Lys Asp Lys Asp Phe Leu Asp
           595                 600                 605
   aat gag gag aac gag gac att ctt gag gac att gtc ctc acc ctt acg      1872
   Asn Glu Glu Asn Glu Asp Ile Leu Glu Asp Ile Val Leu Thr Leu Thr
       610                 615                 620
   ttg ttt gaa gat agg gag atg att gaa gaa cgc ttg aaa act tac gct        1920
   Leu Phe Glu Asp Arg Glu Met Ile Glu Glu Arg Leu Lys Thr Tyr Ala
   625                 630                 635                 640
   cat ctc ttc gac gac aaa gtc atg aaa cag ctc aag agg cgc cga tat        1968
   His Leu Phe Asp Asp Lys Val Met Lys Gln Leu Lys Arg Arg Arg Tyr
                   645                 650                 655
   aca gga tgg ggg cgg ctg tca aga aaa ctg atc aat ggg atc cga gac        2016
   Thr Gly Trp Gly Arg Leu Ser Arg Lys Leu Ile Asn Gly Ile Arg Asp
               660                 665                 670
   aag cag agt gga aag aca atc ctg gat ttt ctt aag tcc gat gga ttt        2064
   Lys Gln Ser Gly Lys Thr Ile Leu Asp Phe Leu Lys Ser Asp Gly Phe
           675                 680                 685
   gcc aac cgg aac ttc atg cag ttg atc cat gat gac tct ctc acc ttt        2112
   Ala Asn Arg Asn Phe Met Gln Leu Ile His Asp Asp Ser Leu Thr Phe
       690                 695                 700
   aag gag gac atc cag aaa gca caa gtt tct ggc cag ggg gac agt ctt        2160
   Lys Glu Asp Ile Gln Lys Ala Gln Val Ser Gly Gln Gly Asp Ser Leu
   705                 710                 715                 720
   cac gag cac atc gct aat ctt gca ggt agc cca gct atc aaa aag gga        2208
   His Glu His Ile Ala Asn Leu Ala Gly Ser Pro Ala Ile Lys Lys Gly
                   725                 730                 735
   ata ctg cag acc gtt aag gtc gtg gat gaa ctc gtc aaa gta atg gga        2256
   Ile Leu Gln Thr Val Lys Val Val Asp Glu Leu Val Lys Val Met Gly
               740                 745                 750
   agg cat aag ccc gag aat atc gtt atc gag atg gcc cga gag aac caa        2304
   Arg His Lys Pro Glu Asn Ile Val Ile Glu Met Ala Arg Glu Asn Gln
           755                 760                 765
   act acc cag aag gga cag aag aac agt agg gaa agg atg aag agg att        2352
   Thr Thr Gln Lys Gly Gln Lys Asn Ser Arg Glu Arg Met Lys Arg Ile
       770                 775                 780
   gaa gag ggt ata aaa gaa ctg ggg tcc caa atc ctt aag gaa cac cca        2400
   Glu Glu Gly Ile Lys Glu Leu Gly Ser Gln Ile Leu Lys Glu His Pro
   785                 790                 795                 800
   gtt gaa aac acc cag ctt cag aat gag aag ctc tac ctg tac tac ctg        2448
   Val Glu Asn Thr Gln Leu Gln Asn Glu Lys Leu Tyr Leu Tyr Tyr Leu
                   805                 810                 815
   cag aac ggc agg gac atg tac gtg gat cag gaa ctg gac atc aat cgg        2496
   Gln Asn Gly Arg Asp Met Tyr Val Asp Gln Glu Leu Asp Ile Asn Arg
               820                 825                 830
   ctc tcc gac tac gac gtg gat cat atc gtg ccc cag tct ttt ctc aaa        2544
   Leu Ser Asp Tyr Asp Val Asp His Ile Val Pro Gln Ser Phe Leu Lys
           835                 840                 845
   gat gat tct att gat aat aaa gtg ttg aca aga tcc gat aaa aat aga        2592
   Asp Asp Ser Ile Asp Asn Lys Val Leu Thr Arg Ser Asp Lys Asn Arg
       850                 855                 860
   ggg aag agt gat aac gtc ccc tca gaa gaa gtt gtc aag aaa atg aaa        2640
   Gly Lys Ser Asp Asn Val Pro Ser Glu Glu Val Val Lys Lys Met Lys
   865                 870                 875                 880
   aat tat tgg cgg cag ctg ctg aac gcc aaa ctg atc aca caa cgg aag      2688
   Asn Tyr Trp Arg Gln Leu Leu Asn Ala Lys Leu Ile Thr Gln Arg Lys
                   885                 890                 895
   ttc gat aat ctg act aag gct gaa cga ggt ggc ctg tct gag ttg gat      2736
   Phe Asp Asn Leu Thr Lys Ala Glu Arg Gly Gly Leu Ser Glu Leu Asp
               900                 905                 910
   aaa gcc ggc ttc atc aaa agg cag ctt gtt gag aca cgc cag atc acc      2784
   Lys Ala Gly Phe Ile Lys Arg Gln Leu Val Glu Thr Arg Gln Ile Thr
           915                 920                 925
   aag cac gtg gcc caa att ctc gat tca cgc atg aac acc aag tac gat      2832
   Lys His Val Ala Gln Ile Leu Asp Ser Arg Met Asn Thr Lys Tyr Asp
       930                 935                 940
   gaa aat gac aaa ctg att cga gag gtg aaa gtt att act ctg aag tct      2880
   Glu Asn Asp Lys Leu Ile Arg Glu Val Lys Val Ile Thr Leu Lys Ser
   945                 950                 955                 960
   aag ctg gtc tca gat ttc aga aag gac ttt cag ttt tat aag gtg aga      2928
   Lys Leu Val Ser Asp Phe Arg Lys Asp Phe Gln Phe Tyr Lys Val Arg
                   965                 970                 975
   gag atc aac aat tac cac cat gcg cat gat gcc tac ctg aat gca gtg      2976
   Glu Ile Asn Asn Tyr His His Ala His Asp Ala Tyr Leu Asn Ala Val
               980                 985                 990
   gta ggc act gca ctt atc aaa aaa  tat ccc aag ctt gaa  tct gaa ttt    3024
   Val Gly Thr Ala Leu Ile Lys Lys  Tyr Pro Lys Leu Glu  Ser Glu Phe
           995                 1000                1005
   gtt tac  gga gac tat aaa gtg  tac gat gtt agg aaa  atg atc gca      3069
   Val Tyr  Gly Asp Tyr Lys Val  Tyr Asp Val Arg Lys  Met Ile Ala
       1010                1015                1020
   aag tct  gag cag gaa ata ggc  aag gcc acc gct aag  tac ttc ttt      3114
   Lys Ser  Glu Gln Glu Ile Gly  Lys Ala Thr Ala Lys  Tyr Phe Phe
       1025                1030                1035
   tac agc  aat att atg aat ttt  ttc aag acc gag att  aca ctg gcc      3159
   Tyr Ser  Asn Ile Met Asn Phe  Phe Lys Thr Glu Ile  Thr Leu Ala
       1040                1045                1050
   aat gga  gag att cgg aag cga  cca ctt atc gaa aca  aac gga gaa      3204
   Asn Gly  Glu Ile Arg Lys Arg  Pro Leu Ile Glu Thr  Asn Gly Glu
       1055                1060                1065
   aca gga  gaa atc gtg tgg gac  aag ggt agg gat ttc  gcg aca gtc      3249
   Thr Gly  Glu Ile Val Trp Asp  Lys Gly Arg Asp Phe  Ala Thr Val
       1070                1075                1080
   cgg aag  gtc ctg tcc atg ccg  cag gtg aac atc gtt  aaa aag acc      3294
   Arg Lys  Val Leu Ser Met Pro  Gln Val Asn Ile Val  Lys Lys Thr
       1085                1090                1095
   gaa gta  cag acc gga ggc ttc  tcc aag gaa agt atc  ctc ccg aaa      3339
   Glu Val  Gln Thr Gly Gly Phe  Ser Lys Glu Ser Ile  Leu Pro Lys
       1100                1105                1110
   agg aac  agc gac aag ctg atc  gca cgc aaa aaa gat  tgg gac ccc        3384
   Arg Asn  Ser Asp Lys Leu Ile  Ala Arg Lys Lys Asp  Trp Asp Pro
       1115                 1120                 1125
   aag aaa  tac ggc gga ttc gat  tct cct aca gtc gct  tac agt gta        3429
   Lys Lys  Tyr Gly Gly Phe Asp  Ser Pro Thr Val Ala  Tyr Ser Val
       1130                 1135                 1140
   ctg gtt  gtg gcc aaa gtg gag  aaa ggg aag tct aaa  aaa ctc aaa        3474
   Leu Val  Val Ala Lys Val Glu  Lys Gly Lys Ser Lys  Lys Leu Lys
       1145                 1150                 1155
   agc gtc  aag gaa ctg ctg ggc  atc aca atc atg gag  cga tca agc        3519
   Ser Val  Lys Glu Leu Leu Gly  Ile Thr Ile Met Glu  Arg Ser Ser
       1160                 1165                 1170
   ttc gaa  aaa aac ccc atc gac  ttt ctc gag gcg aaa  gga tat aaa        3564
   Phe Glu  Lys Asn Pro Ile Asp  Phe Leu Glu Ala Lys  Gly Tyr Lys
       1175                 1180                 1185
   gag gtc  aaa aaa gac ctc atc  att aag ctt ccc aag  tac tct ctc        3609
   Glu Val  Lys Lys Asp Leu Ile  Ile Lys Leu Pro Lys  Tyr Ser Leu
       1190                 1195                 1200
   ttt gag  ctt gaa aac ggc cgg  aaa cga atg ctc gct  agt gcg ggc        3654
   Phe Glu  Leu Glu Asn Gly Arg  Lys Arg Met Leu Ala  Ser Ala Gly
       1205                 1210                 1215
   gag ctg  cag aaa ggt aac gag  ctg gca ctg ccc tct  aaa tac gtt        3699
   Glu Leu  Gln Lys Gly Asn Glu  Leu Ala Leu Pro Ser  Lys Tyr Val
       1220                 1225                 1230
   aat ttc  ttg tat ctg gcc agc  cac tat gaa aag ctc  aaa ggg tct        3744
   Asn Phe  Leu Tyr Leu Ala Ser  His Tyr Glu Lys Leu  Lys Gly Ser
       1235                 1240                 1245
   ccc gaa  gat aat gag cag aag  cag ctg ttc gtg gaa  caa cac aaa        3789
   Pro Glu  Asp Asn Glu Gln Lys  Gln Leu Phe Val Glu  Gln His Lys
       1250                 1255                 1260
   cac tac  ctt gat gag atc atc  gag caa ata agc gaa  ttc tcc aaa        3834
   His Tyr  Leu Asp Glu Ile Ile  Glu Gln Ile Ser Glu  Phe Ser Lys
       1265                 1270                 1275
   aga gtg  atc ctc gcc gac gct  aac ctc gat aag gtg  ctt tct gct        3879
   Arg Val  Ile Leu Ala Asp Ala  Asn Leu Asp Lys Val  Leu Ser Ala
       1280                 1285                 1290
   tac aat  aag cac agg gat aag  ccc atc agg gag cag  gca gaa aac        3924
   Tyr Asn  Lys His Arg Asp Lys  Pro Ile Arg Glu Gln  Ala Glu Asn
       1295                 1300                 1305
   att atc  cac ttg ttt act ctg  acc aac ttg ggc gcg  cct gca gcc        3969
   Ile Ile  His Leu Phe Thr Leu  Thr Asn Leu Gly Ala  Pro Ala Ala
       1310                 1315                 1320
   ttc aag  tac ttc gac acc acc  ata gac aga aag cgg  tac acc tct        4014
   Phe Lys  Tyr Phe Asp Thr Thr  Ile Asp Arg Lys Arg  Tyr Thr Ser
       1325                 1330                 1335
   aca aag  gag gtc ctg gac gcc  aca ctg att cat cag  tca att acg        4059
   Thr Lys  Glu Val Leu Asp Ala  Thr Leu Ile His Gln  Ser Ile Thr
       1340                 1345                 1350
   ggg ctc  tat gaa aca aga atc  gac ctc tct cag ctc  ggt gga gac        4104
   Gly Leu  Tyr Glu Thr Arg Ile  Asp Leu Ser Gln Leu  Gly Gly Asp
       1355                 1360                 1365
   agc agg  gct gac                                                      4116
   Ser Arg  Ala Asp
       1370
<210> 4
   <211> 1372
   <212> PRT
   <213> Artificial Sequence
<220>
   <223> Synthetic Construct
<400> 4
   Met Asp Lys Lys Tyr Ser Ile Gly Leu Asp Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
   180 185 190
   Asn Gln Leu Phe Glu Glu Asn Pro Ile Asn Ala Ser Gly Val Asp Ala
   195 200 205
   Lys Ala Ile Leu Ser Ala Arg Leu Ser Lys Ser Arg Arg Leu Glu Asn
   210 215 220
   Leu Ile Ala Gln Leu Pro Gly Glu Lys Lys Asn Gly Leu Phe Gly Asn
   225 230 235 240
   Leu Ile Ala Leu Ser Leu Gly Leu Thr Pro Asn Phe Lys Ser Asn Phe
   245 250 255
   Asp Leu Ala Glu Asp Ala Lys Leu Gln Leu Ser Lys Asp Thr Tyr Asp
   260 265 270
   Asp Asp Leu Asp Asn Leu Leu Ala Gln Ile Gly Asp Gln Tyr Ala Asp
   275 280 285
   Leu Phe Leu Ala Ala Lys Asn Leu Ser Asp Ala Ile Leu Leu Ser Asp
   290 295 300
   Ile Leu Arg Val Asn Thr Glu Ile Thr Lys Ala Pro Leu Ser Ala Ser
   305 310 315 320
   Met Ile Lys Arg Tyr Asp Glu His His Gln Asp Leu Thr Leu Leu Lys
   325 330 335
   Ala Leu Val Arg Gln Gln Leu Pro Glu Lys Tyr Lys Glu Ile Phe Phe
   340 345 350
   Asp Gln Ser Lys Asn Gly Tyr Ala Gly Tyr Ile Asp Gly Gly Ala Ser
   355 360 365
   Gln Glu Glu Phe Tyr Lys Phe Ile Lys Pro Ile Leu Glu Lys Met Asp
   370 375 380
   Gly Thr Glu Glu Leu Leu Val Lys Leu Asn Arg Glu Asp Leu Leu Arg
   385 390 395 400
   Lys Gln Arg Thr Phe Asp Asn Gly Ser Ile Pro His Gln Ile His Leu
   405 410 415
   Gly Glu Leu His Ala Ile Leu Arg Arg Gln Glu Asp Phe Tyr Pro Phe
   420 425 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Thr Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Arg Glu Met Ile Glu Glu Arg Leu Lys Thr Tyr Ala
   625 630 635 640
   His Leu Phe Asp Asp Lys Val Met Lys Gln Leu Lys Arg Arg Arg Tyr
   645 650 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile His Asp Asp Ser Leu Thr Phe
   690 695 700
   Lys Glu Asp Ile Gln Lys Ala Gln Val Ser Gly Gln Gly Asp Ser Leu
   705 710 715 720
   His Glu His Ile Ala Asn Leu Ala Gly Ser Pro Ala Ile Lys Lys Gly
   725 730 735
   Ile Leu Gln Thr Val Lys Val Val Asp Glu Leu Val Lys Val Met Gly
   740 745 750
   Arg His Lys Pro Glu Asn Ile Val Ile Glu Met Ala Arg Glu Asn Gln
   755 760 765
   Thr Thr Gln Lys Gly Gln Lys Asn Ser Arg Glu Arg Met Lys Arg Ile
   770 775 780
   Glu Glu Gly Ile Lys Glu Leu Gly Ser Gln Ile Leu Lys Glu His Pro
   785 790 795 800
   Val Glu Asn Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp His Ile Val Pro Gln Ser Phe Leu Lys
   835 840 845
   Asp Asp Ser Ile Asp Asn Lys Val Leu Thr Arg Ser Asp Lys Asn Arg
   850 855 860
   Gly Lys Ser Asp Asn Val Pro Ser Glu Glu Val Val Lys Lys Met Lys
   865 870 875 880
   Asn Tyr Trp Arg Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
   900 905 910
   Lys Ala Gly Phe Ile Lys Arg Gln Leu Val Glu Thr Arg Gln Ile Thr
   915 920 925
   Lys His Val Ala Gln Ile Leu Asp Ser Arg Met Asn Thr Lys Tyr Asp
   930 935 940
   Glu Asn Asp Lys Leu Ile Arg Glu Val Lys Val Ile Thr Leu Lys Ser
   945 950 955 960
   Lys Leu Val Ser Asp Phe Arg Lys Asp Phe Gln Phe Tyr Lys Val Arg
   965 970 975
   Glu Ile Asn Asn Tyr His His Ala His Asp Ala Tyr Leu Asn Ala Val
   980 985 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Val Arg Lys Met Ile Ala
   1010 1015 1020
   Lys Ser Glu Gln Glu Ile Gly Lys Ala Thr Ala Lys Tyr Phe Phe
   1025 1030 1035
   Tyr Ser Asn Ile Met Asn Phe Phe Lys Thr Glu Ile Thr Leu Ala
   1040 1045 1050
   Asn Gly Glu Ile Arg Lys Arg Pro Leu Ile Glu Thr Asn Gly Glu
   1055 1060 1065
   Thr Gly Glu Ile Val Trp Asp Lys Gly Arg Asp Phe Ala Thr Val
   1070 1075 1080
   Arg Lys Val Leu Ser Met Pro Gln Val Asn Ile Val Lys Lys Thr
   1085 1090 1095
   Glu Val Gln Thr Gly Gly Phe Ser Lys Glu Ser Ile Leu Pro Lys
   1100 1105 1110
   Arg Asn Ser Asp Lys Leu Ile Ala Arg Lys Lys Asp Trp Asp Pro
   1115 1120 1125
   Lys Lys Tyr Gly Gly Phe Asp Ser Pro Thr Val Ala Tyr Ser Val
   1130 1135 1140
   Leu Val Val Ala Lys Val Glu Lys Gly Lys Ser Lys Lys Leu Lys
   1145 1150 1155
   Ser Val Lys Glu Leu Leu Gly Ile Thr Ile Met Glu Arg Ser Ser
   1160 1165 1170
   Phe Glu Lys Asn Pro Ile Asp Phe Leu Glu Ala Lys Gly Tyr Lys
   1175 1180 1185
   Glu Val Lys Lys Asp Leu Ile Ile Lys Leu Pro Lys Tyr Ser Leu
   1190 1195 1200
   Phe Glu Leu Glu Asn Gly Arg Lys Arg Met Leu Ala Ser Ala Gly
   1205 1210 1215
   Glu Leu Gln Lys Gly Asn Glu Leu Ala Leu Pro Ser Lys Tyr Val
   1220 1225 1230
   Asn Phe Leu Tyr Leu Ala Ser His Tyr Glu Lys Leu Lys Gly Ser
   1235 1240 1245
   Pro Glu Asp Asn Glu Gln Lys Gln Leu Phe Val Glu Gln His Lys
   1250 1255 1260
   His Tyr Leu Asp Glu Ile Ile Glu Gln Ile Ser Glu Phe Ser Lys
   1265 1270 1275
   Arg Val Ile Leu Ala Asp Ala Asn Leu Asp Lys Val Leu Ser Ala
   1280 1285 1290
   Tyr Asn Lys His Arg Asp Lys Pro Ile Arg Glu Gln Ala Glu Asn
   1295 1300 1305
   Ile Ile His Leu Phe Thr Leu Thr Asn Leu Gly Ala Pro Ala Ala
   1310 1315 1320
   Phe Lys Tyr Phe Asp Thr Thr Ile Asp Arg Lys Arg Tyr Thr Ser
   1325 1330 1335
   Thr Lys Glu Val Leu Asp Ala Thr Leu Ile His Gln Ser Ile Thr
   1340 1345 1350
   Gly Leu Tyr Glu Thr Arg Ile Asp Leu Ser Gln Leu Gly Gly Asp
   1355 1360 1365
   Ser Arg Ala Asp
   1370
<210> 5
   <211> 252
   <212> DNA
   <213> Artificial Sequence
<220>
   <223> PBS2-derived Ugi CDS optimized for eucaryotic cell expression
<220>
   <221> CDS
   <222> (1)..(252)
<400> 5
   atg acc aac ctt tcc gac atc ata gag aag gaa aca ggc aaa cag ttg        48
   Met Thr Asn Leu Ser Asp Ile Ile Glu Lys Glu Thr Gly Lys Gln Leu
   1               5                   10                  15
   gtc atc caa gag tcg ata ctc atg ctt cct gaa gaa gtt gag gag gtc        96
   Val Ile Gln Glu Ser Ile Leu Met Leu Pro Glu Glu Val Glu Glu Val
               20                  25                  30
   att ggg aat aag ccg gaa agt gac att ctc gta cac act gcg tat gat       144
   Ile Gly Asn Lys Pro Glu Ser Asp Ile Leu Val His Thr Ala Tyr Asp
           35                  40                  45
   gag agc acc gat gag aac gtg atg ctg ctc acg tca gat gcc cca gag       192
   Glu Ser Thr Asp Glu Asn Val Met Leu Leu Thr Ser Asp Ala Pro Glu
       50                  55                  60
   tac aaa ccc tgg gct ctg gtg att cag gac tct aat gga gag aac aag       240
   Tyr Lys Pro Trp Ala Leu Val Ile Gln Asp Ser Asn Gly Glu Asn Lys
   65                  70                  75                  80
   atc aag atg cta                                                       252
   Ile Lys Met Leu
<210> 6
   <211> 84
   <212> PRT
   <213> Artificial Sequence
<220>
   <223> Synthetic Construct
<400> 6
   Met Thr Asn Leu Ser Asp Ile Ile Glu Lys Glu Thr Gly Lys Gln Leu
   1               5                   10                  15
   Val Ile Gln Glu Ser Ile Leu Met Leu Pro Glu Glu Val Glu Glu Val
               20                  25                  30
   Ile Gly Asn Lys Pro Glu Ser Asp Ile Leu Val His Thr Ala Tyr Asp
           35                  40                  45
   Glu Ser Thr Asp Glu Asn Val Met Leu Leu Thr Ser Asp Ala Pro Glu
       50                  55                  60
   Tyr Lys Pro Trp Ala Leu Val Ile Gln Asp Ser Asn Gly Glu Asn Lys
   65                  70                  75                  80
   Ile Lys Met Leu
<210> 7
   <211> 20
   <212> DNA
   <213> Cricetulus griseus
<400> 7
   ccgagatgtc atgaaagaga 20
<210> 8
   <211> 20
   <212> DNA
   <213> Cricetulus griseus
<400> 8
   ccatgacgga atcggtcggc 20
<210> 9
   <211> 83
   <212> DNA
   <213> Streptococcus pyogenes
<400> 9
   gttttagagc tagaaatagc aagttaaaat aaggctagtc cgttatcaac ttgaaaaagt 60
   ggcaccgagt cggtggtgct ttt 83
<210> 10
   <211> 229
   <212> DNA
   <213> Homo sapiens
<400> 10
   aattcgaacg ctgacgtcat caacccgctc caaggaatcg cgggcccagt gtcactaggc 60
   gggaacaccc agcgcgcgtg cgccctggca ggaagatggc tgtgagggac aggggagtgg 120
   cgccctgcaa tatttgcatg tcgctatgtg ttctgggaaa tcaccataaa cgtgaaatgt 180
   ctttggattt gggaatctta taagttctgt atgaggacca cagatcccc 229
<210> 11
   <211> 21
   <212> DNA
   <213> Artificial Sequence
<220>
   <223> Nuclear transition signal
<220>
   <221> CDS
   <222> (1)..(21)
<400> 11
   ccc aag aag aag agg aag gtg                                            21
                                 Pro Lys Lys Lys Arg Lys Val
                                 1               5
<210> 12
   <211> 7
   <212> PRT
   <213> Artificial Sequence
<220>
   <223> Synthetic Construct
<400> 12
   Pro Lys Lys Lys Arg Lys Val
   1               5
<210> 13
   <211> 54
   <212> DNA
   <213> Artificial Sequence
<220>
   <223> 2A peptide
<220>
   <221> CDS
   <222> (1)..(54)
<400> 13
   gaa ggc agg gga agc ctt ctg act tgt ggg gat gtg gaa gaa aac cct        48
   Glu Gly Arg Gly Ser Leu Leu Thr Cys Gly Asp Val Glu Glu Asn Pro
   1               5                   10                  15
   ggt cca                                                                54
   Gly Pro
<210> 14
   <211> 18
   <212> PRT
   <213> Artificial Sequence
<220>
   <223> Synthetic Construct
<400> 14
   Glu Gly Arg Gly Ser Leu Leu Thr Cys Gly Asp Val Glu Glu Asn Pro
   1               5                   10                  15
   Gly Pro
<210> 15
   <211> 15
   <212> DNA
   <213> Artificial Sequence
<220>
   <223> GS linker
<220>
   <221> CDS
   <222> (1)..(15)
<400> 15
   ggt gga gga ggt tct                                          15
   Gly Gly Gly Gly Ser
   1               5
<210> 16
   <211> 5
   <212> PRT
   <213> Artificial Sequence
<220>
   <223> Synthetic Construct
<400> 16
   Gly Gly Gly Gly Ser
   1               5
<210> 17
   <211> 122
   <212> DNA
   <213> Artificial Sequence
<220>
   <223> SV40 poly A signal terminator
<400> 17
   aacttgttta ttgcagctta taatggttac aaataaagca atagcatcac aaatttcaca     60
   aataaagcat ttttttcact gcattctagt tgtggtttgt ccaaactcat caatgtatct    120
   ta                                                                   122
<210> 18
   <211> 25
   <212> DNA
   <213> Artificial Sequence
<220>
   <223> PCR forward primer
<400> 18
   ggctacatag agggatcctg tgtca 25
<210> 19
   <211> 25
   <212> DNA
   <213> Artificial Sequence
<220>
   <223> PCR reverse primer
<400> 19
   acagtagctc ttcagtctga taaaa 25
<210> 20
   <211> 19
   <212> RNA
   <213> Francisella novicida
<220>
   <221> misc_structure
   <222> (1)..(19)
   <223> crRNA direct repeat sequence.
<400> 20
   aauuucuacu guuguagau 19
<210> 21
   <211> 20
   <212> DNA
   <213> Homo sapiens
<400> 21
   gagtccgagc agaagaagaa 20
<210> 22
   <211> 20
   <212> DNA
   <213> Homo sapiens
<400> 22
   gagttagagc agaagaagaa 20
<210> 23
   <211> 20
   <212> DNA
   <213> Homo sapiens
<400> 23
   gagtctaagc agaagaagaa 20
<210> 24
   <211> 20
   <212> DNA
   <213> Homo sapiens
<400> 24
   gaggccgagc agaagaaaga 20
<210> 25
   <211> 20
   <212> DNA
   <213> Homo sapiens
<400> 25
   gagtcctagc aggagaagaa 20
<210> 26
   <211> 35
   <212> DNA
   <213> Artificial Sequence
<220>
   <223> PCR primer (EMX 1st primer)
<400> 26
   gtagtctggc tgtcacaggc catactcttc cacat 35
<210> 27
   <211> 35
   <212> DNA
   <213> Artificial Sequence
<220>
   <223> PCR primer (EMX 1st primer)
<400> 27
   gtgggtgacc cacccaagca gcaggctctc cacca 35
<210> 28
   <211> 59
   <212> DNA
   <213> Artificial Sequence
<220>
   <223> PCR primer (EMX 2nd primer)
<400> 28
   tctttcccta cacgacgctc ttccgatcta cttagctgga gtgtggaggc tatcttggc 59
<210> 29
   <211> 59
   <212> DNA
   <213> Artificial Sequence
<220>
   <223> PCR primer (EMX 2nd primer)
<400> 29
   gtgactggag ttcagacgtg tgctcttccg atctggctag ggactggcca gagtccagc 59
<210> 30
   <211> 35
   <212> DNA
   <213> Artificial Sequence
<220>
   <223> PCR primer (EMX-off1 1st primer)
<400> 30
   ctgcccatat ccaccacaag caagttagtc atcaa 35
<210> 31
   <211> 30
   <212> DNA
   <213> Artificial Sequence
<220>
   <223> PCR primer (EMX-off1 1st primer)
<400> 31
   aatcaaaatc tctatgtgtg gggcacaggg 30
<210> 32
   <211> 54
   <212> DNA
   <213> Artificial Sequence
<220>
   <223> PCR primer (EMX-off1 2nd primer)
<400> 32
   tctttcccta cacgacgctc ttccgatctc attggctaga attcagactt caag 54
<210> 33
   <211> 59
   <212> DNA
   <213> Artificial Sequence
<220>
   <223> PCR primer (EMX-off1 2nd primer)
<400> 33
   gtgactggag ttcagacgtg tgctcttccg atctatgagg gagatgtact ctcaagtga 59
<210> 34
   <211> 35
   <212> DNA
   <213> Artificial Sequence
<220>
   <223> PCR primer (EMX-off2 1st primer)
<400> 34
   catgttccct cacccttggc atctacacac tttct 35
<210> 35
   <211> 30
   <212> DNA
   <213> Artificial Sequence
<220>
   <223> PCR primer (EMX-off2 1st primer)
<400> 35
   tagtttaccc tgaggcaata tctgactcca 30
<210> 36
   <211> 54
   <212> DNA
   <213> Artificial Sequence
<220>
   <223> PCR primer (EMX-off2 2nd primer)
<400> 36
   tctttcccta cacgacgctc ttccgatctt cattttcaaa tgcctattga gcgg 54
<210> 37
   <211> 59
   <212> DNA
   <213> Artificial Sequence
<220>
   <223> PCR primer (EMX-off2 2nd primer)
<400> 37
   gtgactggag ttcagacgtg tgctcttccg atctaaggct ccttgccttt acatatagg 59
<210> 38
   <211> 30
   <212> DNA
   <213> Artificial Sequence
<220>
   <223> PCR primer (EMX-off3 1st primer)
<400> 38
   tcacttttgt caattcatgc caccatcagt 30
<210> 39
   <211> 30
   <212> DNA
   <213> Artificial Sequence
<220>
   <223> PCR primer (EMX-off3 1st primer)
<400> 39
   gccacctcca ctctgccagg aataggttca 30
<210> 40
   <211> 54
   <212> DNA
   <213> Artificial Sequence
<220>
   <223> PCR primer (EMX-off3 2nd primer)
<400> 40
   tctttcccta cacgacgctc ttccgatcta tggactgtcc tgtgagcccg tggc 54
<210> 41
   <211> 59
   <212> DNA
   <213> Artificial Sequence
<220>
   <223> PCR primer (EMX-off3 2nd primer)
<400> 41
   gtgactggag ttcagacgtg tgctcttccg atctctcggt ggcctgcaag tggaaagcc 59
<210> 42
   <211> 30
   <212> DNA
   <213> Artificial Sequence
<220>
   <223> PCR primer (EMX-off4 1st primer)
<400> 42
   gggaccactt gaagtgagta aaattatagg 30
<210> 43
   <211> 30
   <212> DNA
   <213> Artificial Sequence
<220>
   <223> PCR primer (EMX-off4 1st primer)
<400> 43
   cccagctgtt gctagcttat ggccagtcct 30
<210> 44
   <211> 54
   <212> DNA
   <213> Artificial Sequence
<220>
   <223> PCR primer (EMX-off4 2nd primer)
<400> 44
   tctttcccta cacgacgctc ttccgatctc actgcctttc gggctagcct ccaa 54
<210> 45
   <211> 59
   <212> DNA
   <213> Artificial Sequence
<220>
   <223> PCR primer (EMX-off4 2nd primer)
<400> 45
   gtgactggag ttcagacgtg tgctcttccg atcttagatg ttaataggtt attggggtg 59
<210> 46
   <211> 18
   <212> PRT
   <213> Cricetulus griseus
<400> 46
   Thr Glu Arg Leu Ala Arg Asp Val Met Lys Glu Met Gly Gly His His
   1               5                   10                  15
   Ile Val
<210> 47
   <211> 55
   <212> DNA
   <213> Cricetulus griseus
<400> 47
   gactgaaaga cttgcccgag atgtcatgaa agagatggga ggccatcaca ttgtg 55
<210> 48
   <211> 29
   <212> DNA
   <213> Cricetulus griseus
<400> 48
   gactgaaaga cttgcccgag atgtcatga 29
<210> 49
   <211> 53
   <212> DNA
   <213> Cricetulus griseus
<400> 49
   gactgaaaga cttgcccgag atgtcatgaa agatggaagg ccatcacatt gtg 53
<210> 50
   <211> 55
   <212> DNA
   <213> Cricetulus griseus
<400> 50
   gactgaaaga cttgcccgag atgtcatgaa agagatggga ggccatcaca ttgtg 55
<210> 51
   <211> 56
   <212> DNA
   <213> Cricetulus griseus
<400> 51
   gactgaaaga cttgcccgag atgtcatgaa agaggatggg aggccatcac attgtg 56
<210> 52
   <211> 45
   <212> DNA
   <213> Cricetulus griseus
<400> 52
   gactgaaaga cttgcctgaa agagatggga ggccatcaca ttgtg 45
<210> 53
   <211> 42
   <212> DNA
   <213> Cricetulus griseus
<400> 53
   gactgaaaga ctttgaaaga gatgggaggc catcacattg tg 42
<210> 54
   <211> 55
   <212> DNA
   <213> Cricetulus griseus
<400> 54
   gactgaaaga cttgtttgag atgtcatgaa agagatggga ggccatcaca ttgtg 55
<210> 55
   <211> 55
   <212> DNA
   <213> Cricetulus griseus
<400> 55
   gactgaaaga cttgcttgag atgtcatgaa agagatggga ggccatcaca ttgtg 55
<210> 56
   <211> 55
   <212> DNA
   <213> Cricetulus griseus
<400> 56
   gactgaaaga cttgcctgag atgtcatgaa agagatggga ggccatcaca ttgtg 55
<210> 57
   <211> 31
   <212> DNA
   <213> Homo sapiens
<400> 57
   ggcctgagtc cgagcagaag aagaagggct c 31
<210> 58
   <211> 31
   <212> DNA
   <213> Homo sapiens
<400> 58
   gacaagagtc taagcagaag aagaagagag c 31
<210> 59
   <211> 31
   <212> DNA
   <213> Homo sapiens
<400> 59
   atgaggaggc cgagcagaag aaagacggcg a 31
<210> 60
   <211> 31
   <212> DNA
   <213> Homo sapiens
<400> 60
   gacctgagtc ctagcaggag aagaagaggc a 31